# EUROPÄISCHE PATENTSCHRIFT

(11) **EP 1 579 007 B1**
(45) Veröffentlichungstag und Bekanntmachung des Hinweises auf die Patenterteilung: **27.05.2015**
(21) Anmeldenummer: 03808254.1
(22) Anmeldetag: 19.12.2003
(51) Int. Cl.: C12Q 1/68

(54) **VERFAHREN UND MITTEL ZUR BESTIMMUNG VON BESTIMMTEN ZUSTÄNDEN BZW. VERÄNDERUNGEN IM UTERUSEPITHEL UND IM EPITHEL ANDERER ORGANE**
METHOD AND MEANS FOR DETERMINING SPECIFIC CONDITIONS OR CHANGES IN THE UTERINE EPITHELIUM AND IN THE EPITHELIUM OF OTHER ORGANS
PROCEDE ET DISPOSITIF DE DETERMINATION DE CERTAINS ETATS OU CHANGEMENTS DE L'EPITHELIUM UTERIN ET DE L'EPITHELIUM D'AUTRES ORGANES

(30) Priorität: 21.12.2002 DE 10260556; 06.06.2003 DE 10325637; 06.06.2003 DE 10325636
(43) Veröffentlichungstag der Anmeldung: 28.09.2005
(73) Patentinhaber: Universität Leipzig, 04109 Leipzig (DE)
(72) Erfinder: ZIMMERMANN, Gerolf, 04207 Leipzig (DE); ALEXANDER, Henry, 04299 Leipzig (DE)
(74) Vertreter: Schreiter, Christoph
(86) Internationale Anmeldenummer: PCT/DE2003/004293
(87) Internationale Veröffentlichungsnummer: WO 2004/058999

(56) Entgegenhaltungen:
- US-B1- 6 194 154
- LAZAR V. ET AL: "EXPRESSION OF HUMAN CHORIONIC GONADOTROPIN BETA SUBUNIT GENES IN SUPERFICIAL AND INVASIVE BLADDER CARCINOMAS" CANCER RESEARCH, BALTIMORE, MD, US, Bd. 55, Nr. 17, 1. September 1995 (1995-09-01), Seiten 3735-3738, XP000608909 ISSN: 0008-5472
- BELLET D. ET AL.: "Malignant Transformation of Nontrophoblastic Cells is Associated with the Expression of Chorionic Gonadotropin beta Genes Normally Transcribed in Trophoblastic Cells" CANCER RESEARCH, BALTIMORE, MD, US, Bd. 57, 1. Februar 1997 (1997-02-01), Seiten 516-523, XP002040116 ISSN: 0008-5472
- LUNDIN M. ET AL: "Tissue expression of human chorionic gonadotropin beta predicts outcome in colorectal cancer: A comparison with serum expression" INTERNATIONAL JOURNAL OF CANCER, Bd. 95, Nr. 1, 20. Januar 2001 (2001-01-20), Seiten 18-22, XP002286298 ISSN: 0020-7136
- HOTAKAINEN K. ET AL.: "The free beta-subunit of human chorionic gonadotropin as a prognostic factor in renal cell carcinoma" BRITISH JOURNAL OF CANCER, Bd. 86, Nr. 2, 21. Januar 2002 (2002-01-21), Seiten 185-189, XP002286299 ISSN: 0007-0920

## Beschreibung

Die Erfindung betrifft Verfahren und Mittel zur Bestimmung von definierten Zuständen bzw. Veränderungen im Uterus. Zustände des Uterusepithel oder Epithelien anderer Organe, die mit der Erfindung insbesondere bestimmt werden sollen, sind die Rezeptivität der Uterusschleimhaut für die Implantation eines Embryos oder neoblastische und tumoröse Veränderungen. Anwendungsgebiet ist die Medizin, insbesondere die Gynäkologie und die Onkologie.

Humanes Choriongonadotropin (hCG) ist ein Hormon, dessen Konzentration während der Schwangerschaften erhöht ist, und bei Schwangerschaftstests nachgewiesen wird. hCG besteht aus zwei Untereinheiten α-hCG und β-hCG in nicht-konvalenter Bindung. Für die Untereinheit α-hCG ist ein Gen bekannt (Chromosom 6q21.1-q 23). Für die Untereinheit β-hCG sind 7 Gene β8, β7, β6, β5, β3, β1 und β2 bekannt (Chromosom 19q13.3).

Während der Schwangerschaft werden durch Trophoblasten der Gebärmutter größere Mengen hCG-Dimer und freie α-hCG-und β-hCG -Moleküle gebildet und in das Blut sezerniert. Aber auch in einigen nicht-trophoblastären Geweben wird hCG bzw. seine Untereinheiten in geringen Mengen exprimiert (2-6). Auch im Blut nichtschwangerer gesunder Menschen werden daher hCG-Konzentrationen von hCG bis 1000 pg/ml und von β-hCG bis 100 pg/ml beobachtet (7,8). Höhere β-hCG-Serumwerte deuten auf einen gonadalen oder nicht-gonadalen Tumor hin und kennzeichnen eine ungünstige Prognose, wie bei Lungen-, Blasen-, Prostata-, Colon-, Nierenzell-und Mammakarzinom beschrieben (5,9-13).

In der Publikation von G.W. Wolkersdörfer et al. "The presence of chorionic gonadotropin β subunit in normal cyclic human endometrium", Molecular Human Reproduction vol.4, no.2 pp. 179-184, (1998) wird gezeigt, dass β-hCG in der Uterusschleimhaut in normalen Zyklus einer Frau vorkommt.

Embryonales trophoblastäres Gewebe exprimiert fast ausschließlich hCG β5, β8 und β3. Diese β-hCG Untereinheiten werden daher auch trophoblastäres β-hCG (tβ-hCG) oder Typ II-β-hCG genannt. hCG β7 und β6- werden nur in geringem Umfang in einigen nicht-trophoblastären Geweben, wie z. B. Mamma, Lunge, Prostata, Skelettmuskulatur, Blase, Colon, Uterus, exprimiert (17). Diese β-hCG Untereinheiten werden daher auch als nicht-trophoblastäres β-hCG oder Typ I-β-hCG bezeichnet.

Während die Untereinheiten des Typ II-β-hCG (β5, β8 und β3) an Position 117 (Exon 3) der Aminosäuresequenz ein Aspartat (Asp, D) enthalten, entält Typ 1-β-hCG (β7 und β6) an Position 117 ein Alanin (Ala, A).

In der Vergangenheit sind verschiedene Studien mit dem Ziel durchgeführt worden, die β-hCG-Transkripte in verschiedenen normalen und neoplastischen Geweben nichttrophoblastärer Herkunft mit semiquantitativer Methode nachzuweisen (5, 11, 12, 18). Diese Methoden zeigen, dass β-hCG in normaler Plazenta (19), gesunden Testes (6), aber auch neoplastischen Testes (20) und neoplastischem Blasengewebe (21) transkribiert wird. In diesen Studien wird jedoch nicht zwischen Typ I-β-hCG und Typ II-β-hCG unterschieden.

In einer Arbeit (9) wird die Anwesenheit von hCG β7 in gesundem und von hCG β8, β5, β3 in malignem Blasengewebe durch spezifische Restriktionsenzyme für die Erkennung einzelner Transkripte nachgewiesen.

Eine weitere Arbeit bestimmt die Überexpression von Typ II-β-hCG (β5, β8, β3) in malignem transformiertem nicht-trophoblastärem Gewebe durch den ermittelten Transformationsindex, bestehend im Verhältnis zwischen der Genexpression von hCG β5, β8, β3 zur Gesamtexpression aller β-hCG-Gene im selben Gewebe. Er wird mit Primern zwischen Exon 2 und Exon 3 erfasst, die die Punktmutation C117 in der C-terminalen Region des βhCG im Exon 3 erkennen (17). Bisher wird diese Punktmutation Asp - Ala in Position 117 der β-hCG-Aminosäureketten im genannten Quotient als diagnostischer Parameter der neoplastischen Transformationen genutzt.

Eine Tumoridentifizierung durch Analyse der Sekretionsprodukte, insbesondere der Nutzung des Typ II-β-hCG als Indikator für eine Krebserkrankung, zeigte eine französische Arbeitsgruppe bereits 1996. Beschrieben wird von Bellet et al. (17), dass die β-Untereinheit von hCG durch vier nicht-allele β-hCG-Gene codiert wird. Zu den wesentlichen Erkenntnissen gehört, dass die maligne Transformation nicht-trophoblastären Gewebes stets mit der Expression von β-hCG-Genen verbunden ist, die normalerweise im Trophoblast transkribiert werden. Die Erforschung der β-hCG-Gene, die durch nicht-trophoblastäres Gewebe exprimiert werden, führt zu dem Ergebnis : normales nicht-trophoblastäres Gewebe exprimiert hauptsächlich β-hCG- Gene vom Typ I (hCG β7, β6), während nach maligner Transformation auch β-hCG- Gene vom Typ II (hCG β5, β8, β3) exprimiert werden.

In US-PS 6,194, 154 wird ein Verfahren zur Bestimmung der malignen Transformation humaner Zellen beschrieben, das die Überexpression von hCG β3, β5, β8 und β9- mRNA in malignen Zellen mit der Expression von hCG β7, β6 in nicht-malignen Zellen vergleicht. Bestimmt wird auch die Steigerung der mRNA-Expression von hCG β3, β5, β8 und β9 im Verhältnis zur Gesamt-β-Genexpression in den malignen Zellen. Weiterhin wird ausgeführt, dass die Punktmutation in der mRNA-Nukleotidsequenz von Position 775 für β5, β8, β3 ein A und für β7, β6 ein C anzeigt und in der Aminosäureposition 117 somit Aspartat (Asp, D) oder Alanin (Ala, A) codiert. Auf dieser Basis baut sich ein Testkit auf, der Verbreitung gefunden hat.

WO 0190344 nimmt Bezug auf den Promotor, Enhancer und andere Regulatoren, die die Expression des Proteins β-hCG im testikulären Karzinom kontrollieren. Weiterhin erfolgen Ausführungen zur Gentherapie unter Einschleusung von Promotorgen-β-hCG- DNA in verschiedene Zellen, z. B. in Liposomen. Das β-hCG-Protein wird in verschiedenen Tumorgeweben als diagnostischer Parameter verwendet.

Aufgabe der Erfindung ist es, ein Verfahren und Mittel zur Bestimmung der Aufnahmebereitschaft der Uterusschleimhaut für die Implantation eines Embryos bereit zu stellen.

Erfindungsgemäß wird die Aufgabe gelöst durch ein Verfahren gemäß Anspruch 1, zur Bestimmung der Aufnahmebereitschaft der Uterusschleimhaut für die Implantation eines Embryos, bei dem mRNA aus einer Blut- und/oder Gewebeprobe isoliert wird und in dieser Probe eine quantitative Messung der Expression oder Überexpression der mRNA von β7-hCG und/oder β6-hCG und/oder einer mRNA, deren cDNA eine Sequenz nach SEQ ID No 7 aufweist, erfolgt, wobei eine Anwesenheit von β7-hCG und/oder β6-hCG und/oder einer mRNA, deren cDNA eine Sequenz nach SEQ ID No. 7 aufweist, ein Indikator für die optimale Implantation darstellt.

Die Aufgabe wird ebenfalls gelöst durch die Verwendung eines Primers gemäß Anspruch 13, eines Kits gemäß Anspruch 14 oder einer cDNA gemäß Anspruch 20 zur Bestimmung der Aufnahmebereitschaft der Uterusschleimhaut für die Implantation eines Embryos.

β7-hCG hat die Gensequenz (cDNA) gemäß SEQ ID No 5 und β6-hCG gemäß SEQ ID No 6. β6e-hCG ist die Konsensussequenz des Typ-I-β-hCG (β6 oder β7), mit einer Gensequenz (cDNA) gemäß SEQ ID NO 7. Das β6e-hCG-Gen wird im Endometrium exprimiert und codiert für ein Protein gemäß SEQ ID No 17 oder SEQ ID No 18.

In einer bevorzugten Variante des erfindungsgemäßen Verfahrens wird als interner Standard die Gesamt-βhCG-mRNA-Genexpression oder die mRNA-Genexpression einzelner oder aller Typ-II-β-hCG-Untereinheiten (β5-hCG, β8-hCG, β3-hCG) gemessen. Die mRNA-Genexpression von β7-hCG- und/oder β6-hCG und/oder β6e-hCG wird zur Auswertung dann zu dem Referenzstandard in Relation gesetzt.

Bevorzugt erfolgt die qunatitative Messung der mRNA-Genexpression mittels quantitativer RT-PCR. In dem bekannten Prozess der RT-PCR wird zunächst mit Hilfe des Enzyms Reverser Transkriptase (RT) basierend auf der isolierten RNA komplementäre DNA (cDNA) synthetisiert. Als Primer für die RT wird ein Oligonucleotid mit einer poly-dT-Sequenz gewählt (oligo-dT). Das oligo-dT ist bevorzugt aus 10 bis 20 Deoxythymidin (dT)-Monomeren aufgebaut. Einzelne cDNAs werden in der anschließenden PCR mit einem sequenzspezifischen Primerpaar amplifiziert.

Die Sequenz mindestens eines Primers wird dabei vorzugsweise so gewählt, dass der Primer mit einer β-hCG-cDNA-Sequenz hybridisiert, die durch die Verbindung von 2 Exons gebildet wird. Durch diese Auswahl wird erreicht, dass durch den Primer nur cDNA, jedoch nicht mögliche in der Probe enthaltene Verunreinigungen an genomischer β-hCG-DNA amplifiziert werden.

Als externer Standard wir bevorzugt eine definierte Menge an mRNA oder auch cDNA von β7-hCG bzw. β5-hCG in einer parallel unter identischen Bedingungen durchgeführten Messung verwendet.

Besonders bevorzugt wird ei PCR als Real-time-PCR durchgeführt. Bekannte Real-time PCR Verfahren sind z.B. die TaqMan, FRET (Fluoreszenz-Resonanz-Energie-Transfer) und Beacon-Verfahren. Durch die Verwendung von Fluoreszenz-markierten Primern kann bei diesem Verfahren vorteilhaft das PCR-Produkt während der PCR quantifiziert werden.

Die Erfindung beansprucht auch die Real time-Messung als one tube-RT-PCR oder die Verwendung anderer Methoden zur quantitativen Erfassung der Expression spezifischer Genkopien neben SYBR Green I, wie zum Beispiel der Einsatz von genspezifischen Oligonukleotiden als Hybridisierungsproben mit unterschiedlichen Farbstoff-oder Fluoreszenzmarker-Anbindung (TaqMan, FRET, Beacon).

In einer besonders bevorzugten Variante des erfindungsgemäßen Verfahrens wird basierend auf der durch die Reverse Transkriptase (RT) erhaltene cDNA in einem ersten PCR-Schritt mit mindestens einem ersten Primerpaar Gesamt-βhCG-cDNA amplifiziert.

Die Amplifikation von Gesamt-βhCG wird dadurch erreicht, dass dieses erste Primerpaar sowohl mit cDNA von Typ-II-β-hCG-Untereinheiten (β5-hCG, β8-hCG, β3- hCG), als auch Typ-I-β-hCG-Untereinheiten (β7 und β6 und β6e) hybridisiert.

In einem anschließenden zweiten PCR-Schritt wird mit mindestens einem dritten Primer die cDNA einzelner oder aller Typ-I-β-hCG-Untereinheiten (β7, β6, β6e) spezifisch amplifiziert. Damit im zweiten Schritt nur Typ-I-β-hCG und nicht Typ-II-β-hCG amplifiziert wird, wird der dritte Primer so gewählt, dass er nur mit cDNA von β7-hCG und β6-hCG und β6e-hCG spezifisch hybridisiert, jedoch nicht mit cDNA von β5-hCG, β8-hCG und β3-hCG.

Bevorzugt wird in dem zweiten PCR-Schritt, d. h. einer sogenannten "nested PCR", zusätzlich mit mindestens einem vierten Primer die cDNA von mindestens einer oder mehreren Typ-II-β-hCG-Untereinheiten (β5-hCG und/oder β8-hCG und/oder β3-hCG) spezifisch amplifiziert. Dazu wird der vierte Primer so gewählt, dass er mit cDNA von β5-hCG, β-hCG und β3-hCG spezifisch hybridisiert, jedoch nicht mit cDNA von β7-hCG und β6-hCG und β6e-hCG.

Die Primer für den zweiten Schritt der PCR können vor oder nach Durchführung des ersten Schritts der PCR zugegeben werden.

Die dritten und vierten Primer sind vorzugsweise mit unterschiedlichen Markermolekülen versehen, die eine Unterscheidung zwischen den PCR-Produkten, die durch die Amplifikation mit dem dritten und vierten Primer gebildet werden, ermöglichen.

Bevorzugt wird als erster Primer des ersten Primerpaars zur Amplifikation von GesamtβhCG ein 10 bis 30 Basenpaar-langes DNA-Oligonukleotid aus Exon 1 des βhCG gewählt. Ein derart bevorzugter Primer hat die Sequenz gemäß SEQ ID NO 1.

Bevorzugt wird als zweiter Primer des ersten Primerpaars zur Amplifikation von Gesamt-βhCG ein 10 bis 30 Basenpaar-langes DNA-Oligonukleotid der komplementären Sequenz von Exon 3 des βhCG gewählt. Ein derart bevorzugter Primer hat die Sequenz gemäß SEQ ID NO 2.

Weitere bevorzugte Primer für das Primerpaar zur Amplifikation von Gesamt-βhCG sind Primer mit Sequenzen gemäß SEQ ID NO 11 und SEQ ID NO 14.

Als dritter Primer zur spezifischen Amplifikation von Typ-I-βhCG wird bevorzugt ein 10 bis 30 Basenpaar-langes DNA-Oligonukleotid aus dem Bereich des β7-hCG gewählt. Ein derart bevorzugter Primer hat die DNA-Sequenz gemäß SEQ ID NO 3. Weitere bevorzugte Primer zur spezifischen Amplifikation von Typ-I-βhCG sind Primer mit Sequenzen gemäß SEQ ID NO 9, SEQ ID NO 10, SEQ ID NO 13 und SEQ ID NO 16.

Als vierter Primer zur spezifischen Amplifikation von Typ-II-βhCG wird bevorzugt ein 10 bis 30 Basenpaar-langes DNA-Oligonukleotid aus dem Bereich des β5-hCG gewählt. Ein derart bevorzugter Primer hat die DNA-Sequenz gemäß SEQ ID NO 4. Weitere bevorzugte Primer zur spezifischen Amplifikation von Typ-II-βhCG sind Primer mit Sequenzen gemäß SEQ ID NO 8, SEQ ID NO 12 und SEQ ID NO 15.

Für die bevorzugte Realttime-PCR ist mindestens einer der Primer fluoreszenzmarkiert. Besonders bevorzugt ist der dritte Primer mit einem solchen Fluoreszenzmarker versehen um eine Quantifizierung der amplifizierten Typ-I-βhCG cDNA während der PCR zu ermöglichen.

Bevorzugt ist auch einer der beiden Primer des ersten Primerpaars und gegebenenfalls der vierte Primer mit Fluoreszenzmarkern versehen, wobei sich jedoch die Marker dieser Primer untereinander und zu Primer 1 in ihren Adsorptions- und/oder Emmissionsspektren unterscheiden.

Durch diese unterschiedlichen Fluoreszenzmarker wird eine parallele Quantifizierung der amplifizierten Typ-I-βhCG cDNA und gegebenenfalls Typ-II-βhCG cDNA während der PCR und ein Vergleich mit der Gesamt-βhCG cDNA möglich.

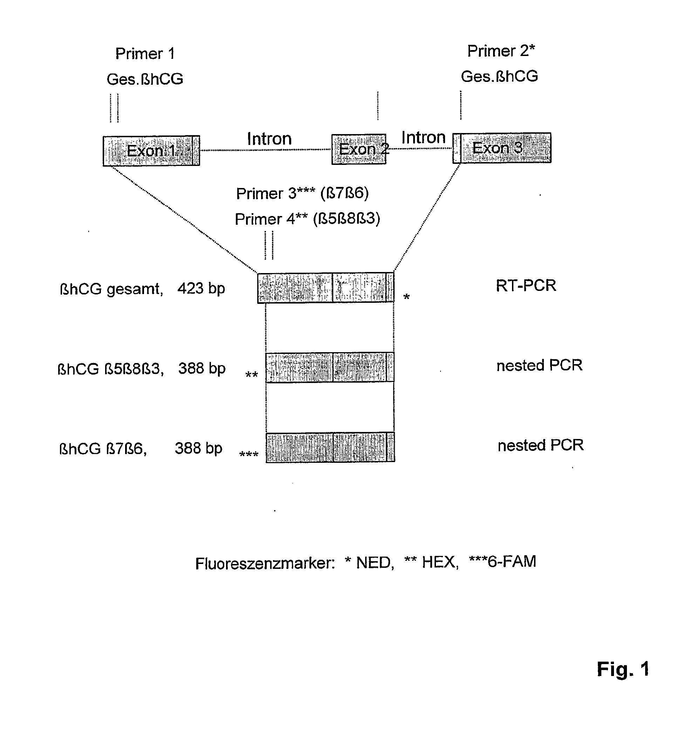

Das erfindungsgemäß Verfahren wird anhand des Flussschemas aus Fig. 1 erläutert. Der in Fig. 1 verwendete Primer 1 (Amplifikation von Gesamt-βhCG) ist nicht markiert, Primer 2 (Amplifikation von Gesamt-βhCG) enthält den Fluoreszenzmarker NED.

Der in Fig. 1 zur Amplifkation von Typ-I-β-hCG (β7, β6, β6e) verwendete Primer 3 ist mit 6-FAM markiert. Primer 4 zur Amplifikation von Typ-II-β-hCG (β5, β8, β3) enthält den Fluoreszenzmarker HEX.

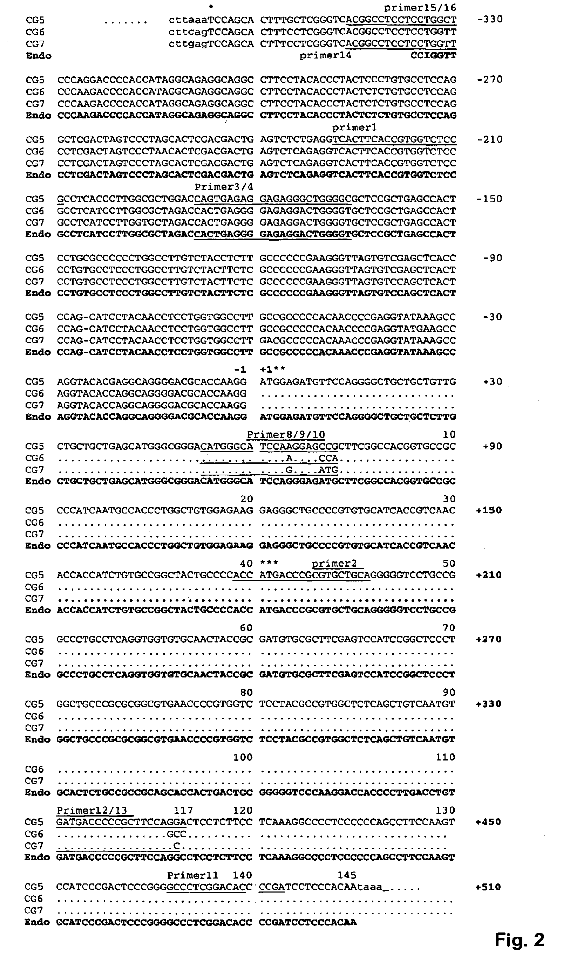

Die Auswahl der Primer ist in Fig. 2 dargestellt.

Fig. 2 zeigt ein Sequenzalignment der Sequenzen β5-hCG ("CG5"), β6-hCG ("CG6"), β7-hCG ("CG7") und das experimentell ermittelte β7-hCG ("Endo"). * bezeichnet den Transkriptionsstart, ** bezeichnet den Translationsstart.

Die Ziffern über den Nukleinsäuresequenzen bezeichnen die Aminosäurepositionen des codierten Proteins. Durch Unterstreichung sind die Sequenzbereich markiert, die mit den Primern hybridisieren.

Die Oligonukleotid-Primerpaare 1 und 2, gemäß SEQ ID No 1 und No 2, 1 und 11, gemäß SEQ ID No 1 und No 11, sowie 14 und 2, gemäß SEQ ID No 14 und No 2 des Sequenzprotokolls wurden derart ausgewählt, dass sie unter Verwendung der Gesamt-RNA und der RT-PCR-Methode in einem ersten Amplifikationsschritt die Summe aller βhCG-Transkripte β5, β8, β3 und auch β7, β6 in gleicher Effizienz darstellen. Diese genannten Primerpaare schließen die βLH-Amplifikation wegen differenter Nukleotidsequenzfolgen aus. Im folgenden nested PCR-Schritt oder unter Verwendung der Real time RT-PCR-Quantifizierungsmethode wird unter Verwendung der Primer 3 und 2, Primer 9 bzw. 10 und 2, Primer 13 und 11 sowie Primer 16 und 12 das Transkript β7, β6, β6e und mit Primer 4 und 2, Primer 8 und 2, Primer 12 und 11 sowie Primer 15 und 2 das Transkript β5, β8, β3 amplifiziert.

Durch den Primer 9 mit der Sequenz gemäß SEQ ID NO 9 wird nur β6-hCG und nicht β7-hCG und β6e-hCG amplifiziert.

Durch den Primer 10 mit der Sequenz gemäß SEQ ID NO 10 wird nur β7-hCG und β6e-hCG amplifiziert und nicht β6-hCG amplifiziert.

Durch den Primer 13 mit der Sequenz gemäß SEQ ID NO 13 wird nur β7-hCG und β6-hCG und nicht β6e-hCG amplifziert.

Durch eine parallele Vervielfältigung von cDNA mit den Primern 9, 10 und 13 (mit den Sequenzen SEQ ID NO 9 und/oder SEQ ID NO 10 und/oder SEQ ID NO 13) kann überraschend zwischen der mRNA-Expression von β7-hCG und β6-hCG und β6e unterschieden werden.

Dazu wird bevorzugt eine RT-PCR durchgeführt, die der oben beschriebenen Methode zur Unterscheidung der Expression von Typ I und Typ II β-hCG entspricht, mit dem Unterschied, dass im zweiten Schritt zwei unterscheidlichlich markierte Primer aus der Gruppe der Sequenzen SEQ ID NO 9, SEQ ID NO 10, SEQ ID NO 13 verwendet werden.

Die Erfindung liefert ein Modell für tumorspezifische Gentranskription speziell eines neuen Promotors βhCG, der nur in verschiedenen Tumorgeweben aktiviert wird, einschließlich, aber nicht begrenzt auf das testikuläre Karzinom. Die Erfindung gibt auch Methoden zur Analyse der Promotorexpression Typ-I-β-hCG-Untereinheiten und Typ-II-β-hCG-Untereinheiten an. Dazu wird eine PCR Primer 15 und 16 (SEQ ID NO 15, SEQ ID NO 16) die mit dem Promoterbereich hybridisieren durchgeführt.

Zur Durchführung des erfindungsgemäßen Verfahrens mittels RT-PCR wird bevorzugt ein Diagnostischer Kit verwendet, der jeweils eine Menge der folgenden Bestandteile enthält:
1. für die cDNA-Synthese :
   a. ) Oligo-dT ;
   b. ) Reverse Transkriptase ;
2. für die PCR :
   c. ) mindestens zwei Primer, die mit cDNA einer oder mehrerer Typ-II-β-hCG-Untereinheiten, Typ-I-β-hCG (β7, β6, eβ6) hybridisieren, wobei mindestens einer der beiden Primer sequenzspezifisch für Typ-I-β-hCG. ist, d. h. nicht mit Typ-II-β-hCG (β5, β8, β3) hybridisiert;
   d. ) eine über 80°C beständige DNA-Polymerase, wie z. B. taq-Polymerase ;
sowie entsprechende Reaktionspuffer.

Zusammensetzungen derartiger Reaktionspuffer sind dem Fachmann bekannt und enthalten üblicherweise RNAse-Inhibitor und für als Bausteine für die Polymerase dNTPs, sowie eine Menge zweiwertiger Kationen, wie Mg2+.

Vorzugsweise enthält der diagnostische Kit eine Menge eines ersten Primerpaars, das sowohl mit cDNA von Typ-II-β-hCG (β5, β8, β3) als auch Typ-I-β-hCG (β7, β6 und β6e) hybridisiert und einen dritten Primer, der sequenzspezifisch für Typ-I-β-hCG ist, also nicht mit Typ-II-β-hCG (β5, β8, β3) hybridisiert.

Bevorzugt wird als erster Primer des ersten Primerpaars zur Amplifikation von Gesamt- βhCG ein 10 bis 30 Basenpaar-langes DNA-Oligonukleotid aus Exon 1 des βhCG gewählt. Ein derart bevorzugter Primer hat die DNA-Sequenz gemäß SEQ ID NO 1.

Bevorzugt wird als zweiter Primer des ersten Primerpaars zur Amplifikation von Gesamt-βhCG ein 10 bis 30 Basenpaar-langes DNA-Oligonukleotid der komplentären Sequenz von Exon 3 des βhCG gewählt. Ein derart bevorzugter Primer hat die DNASequenz gemäß SEQ ID NO 2.

Weitere bevorzugte Primer für das Primerpaar zur Amplifikation von Gesamt-βhCG sind Primer mit Sequenzen gemäß SEQ ID NO 11 und SEQ ID NO 14.

Als dritter Primer zur spezifischen Amplifikation von Typ-I-βhCG wird bevorzugt ein 10 bis 30 Basenpaar-langes DNA-Oligonukleotid aus dem Bereich des β7- hCG gewählt. Ein derart bevorzugter Primer hat die DNA-Sequenz gemäß SEQ ID NO 3.

In einer Bevorzugten Ausführungsform enthält der diagnostische Kit eine Menge eines vierten Primers, der mit cDNA von Typ-I-β-hCG spezifisch hybridisiert, jedoch nicht mit cDNA von Typ-II-β-hCG. Weitere bevorzugte Primer zur spezifischen Amplifikation von Typ-I-βhCG sind Primer mit Sequenzen gemäß SEQ ID NO 9, SEQ ID NO 10, SEQ ID NO 13 und SEQ ID NO 16.

Als vierter Primer zur spezifischen Amplifikation von Typ-II-βhCG wird bevorzugt ein 10 bis 30 Basenpaar-langes DNA-Oligonukleotid aus dem Bereich des β5- hCG gewählt. Ein derart bevorzugter Primer hat die DNA-Sequenz gemäß SEQ ID NO 4. Weitere bevorzugte Primer zur spezifischen Amplifikation von Typ-II-βhCG sind Primer mit Sequenzen gemäß SEQ ID NO 8, SEQ ID NO 12 und SEQ ID NO 15.

Bevorzugt ist mindestens einer der Primer fluoreszenzmarkiert. Dies ermöglicht die Durchführung einer Real-time-PCR.

Besonders bevorzugt sind ein Primer des ersten Primerpaars, der dritte Primer und gegebenenfalls der vierte Primer mit Fluoreszenzmarkern, die sich in ihren Adsorptions- und/oder Emmissionspektren zueinander unterscheiden, zu versehen.

Bestandteil der Erfindung sind auch die Primer mit den Sequenzen gemäß SEQ ID NO 3 und SEQ ID NO 4 sowie SEQ ID NO 8 bis SEQ ID NO 16.

Das Verfahren wird erfindungsgemäß zur Bestimmung von definierten Zuständen oder Veränderungen im Uterus verwendet.

Mit dem erfindungsgemäßen Verfahren kann die Aufnahmebereitschaft der Uterusschleimhaut für die Implantation eines Embryos festgestellt werden.

Eine bevorzugte Verwendung des Verfahrens ist die Verwendung zur Diagnostik der Rezeptivität der Uterusschleimhaut (Implantationsdiagnostik).

Unter der Diagnostik der Rezeptivität der Uterusschleimhaut wird im Sinne der vorliegenden Erfindung das Feststellen von optimalen Implantationsbedingungen, d. h. das Erkennen der Möglichkeit verstanden, dass für eine befruchtete Eizelle in der Uterüsschleimhaut optimale Bedingungen bestehen, sich einzubetten und dort nachfolgend zu wachsen.

Der Erfindung liegt die wissenschaftliche Erkenntnis zugrunde, dass die Höhe der Expression der Gene von Typ-I-β-hCG (β7, β6, eβ6) im normalen sekretorischen Epithelium der Uterusschleimhaut (Endometrium) oder in den mononukleären Zellen des peripheren Blutes ein zuverlässiger Indikator für eine mögliche erfolgreiche Implantation darstellen. Umso höher die Expression umso besser sind die Chancen für eine erfolgreiche Implantation einer befruchteten Eizelle oder eines Embryos.

Der erfindungsgemäßen Verwendung liegt die wissenschaftliche Erkenntnis zugrunde, dass ein zuverlässiger Indikator für eine mögliche erfolgreiche Implantation die Bewertung des Anteils der exprimierten 5'-nichttranslatierenden Promotorsequenzen des βhCG (Exon 1) von βhCG-Gen β7, β6 absolut oder relativ zu β5, β8, β3 darstellt.

Die Gene hCG β7 und β6 des Genclusters werden hauptsächlich im normalen sekretorischen Epithelium der Uterusschleimhaut exprimiert. Die Gene hCG β5, β8, β3 des Genclusters werden im normalen Trophoblast und im karzinom-transformierten Epithel exprimiert. Lymphozyten (CD3), Natural Killer Zellen und Monozyten (CD14) exprimieren-bei Normalpersonen hCG β5.

Zur erfindungsgemäßen Diagnostik der Rezeptivität der Uterusschleimhaut ist die Bestimmung der Expression von hCG und des allelen Gens β7 erforderlich. Es wurde erkannt, dass der Gehalt an β6- und β7-hCG von im körpereigenen epithelialen

Gewebe oder Blutzellen den Erfolg einer Implantation wesentlich bestimmt, und dass deshalb die Kenntnis der Menge an hCG β7 und β6 absolut oder relativ betrachtet in Kenntnis des Quotienten aus hCG β7, β6 als Zähler und hCG β5, β8, β3 als Nenner Aufschluss über den erfolgversprechenden Implantationsmoment gibt.

Zur Bestimmung des hCG β7, β6, β6e und des hCG β5, β8, Anteils ist die quantitative RT-PCR geeignet.

Zur Diagnostik der Rezeptivität der Uterusschleimhaut wird einer Patientin vorzugsweise Gewebe vom Endometrium oder von der Zervixschleimhaut oder Peripherblut entnommen wird und die Analyse der mRNA-Expression in dieser Blut- oder Gewebeprobe mit dem erfindungsgemäßen verfahren bestimmt. Aus der Höhe der ermittelten mRNA-Expression von β7-hCG und/oder β6-hCG und/oder eß6-hCG können dann Rückschlüsse auf die Aufnahmebereitschaft der Gebärmutter für einen Embryo im aktuellen oder Folgezyklus getroffen werden.

Dazu werden bevorzugt 4 bis 6 Tage nach der Ovulation Zellen mit einem Minikatheter aus der Gebärmutterhöhle, mit einem Wattebausch aus dem Zervikalkanal oder mit einem Holzspatel von der Mundschleimhaut Zellen gewonnen bzw. peripheres EDTA- bzw. Heparinblut entnommen. Aus den aufgenommenen Zellen wird die mRNA βhCG isoliert, durch RT-PCR cDNA hergestellt, die cDNA amplifiziert und quantitativ r. bestimmt.

Die Herstellung und Amplifikation der cDNA aus der mRNA erfolgt vorzugsweise durch Real time-Messung in einer one tube-RT-PCR. Alternativ werden andere Methoden zur erfindungsgemäßen quantitativen Erfassung der Expression von spezifischer Genkopien verwendet, vorzugsweise unter Einsatz von genspezifischen Oligonukleotiden als Hybridisierungsproben mit unterschiedlichen Farbstoff-oder Fluoreszenzmarker-Anbindung (TaqMan, FRET, Beacon).

Ein positiver Nachweis der mRNA von β6-hCG, β7-hCG oder β6e-hCG zeigt an, dass sich das Endometrium in Richtung einer Implantationsreife differenziert.

Eine weitere bevorzugte Verwendung des Verfahrens ist die Verwendung zur retrospektiven Diagnostik der Rezeptivität der Uterusschleimhaut. Unter retrospektiven Implantationsdiagnostik wird im Sinne der vorliegenden Erfindung verstanden, dass im vergangenen Zyklus optimale Implantationsbedingungen bestanden haben. Über die Aussage der Implantationsbedingungen im vergangenen Zyklus lassen sich Prognosen über die Implantationsbedingungen, d. h. die Aufnahmebereitschaft der Gebärmutter für eine befruchtete Eizelle oder einen Embryo, im Folgezyklus machen.

Zur retrospektiven Diagnostik der Rezeptivität der Uterusschleimhaut wird prinzipiell, wie zur vorbereitenden Implantationsdiagnostik verfahren, mit dem Unterschied, dass die Analyse der β6- und β7-hCG-Expresssion in einer Probe von Menstrualblut erfolgt. Im Menstrualblut sind ausreichend Zellen des Endometrium vorhanden, die eine Analyse ermöglichen.

Der Vorteil der Analyse im Menstrualblut gegenüber der zuvor beschriebenen Methode, liegt darin, dass sie nicht invasiv ist. Es muss weder Peripherblut noch eine Gewebeprobe aus der Gebärmutter entnommen werden.

Eine weiteres Beispiel ist die Verwendung des Verfahrens zur Tumordiagnostik.

Dieser Verwendung liegt die wissenschaftliche Erkenntnis zugrunde, dass ein zuverlässiger Indikator für das Vorhandensein und das Wachstum von Tumorzellen die Bewertung des Anteils exprimierter 5'-nichttranslatierenden Promotorsequen zen des βhCG (Exon 1) von βhCG-Gen β7, β6 zu β5, β8, β3 darstellt, die sich in diesem Genausschnitt in einer Vielzahl von Nukleotiddifferenzen unterscheiden.

Im Unterschied zur Mutation eines einzigen Nukleotides im Codons 117 des vorbeschriebenen C117-Assays (Exon 3) differieren die βhCG-Gene β7, β6 zu denen von β5, β8, β3 in diesem Genabschnitt des βhCG-Promotorgens (Exon 1) in einer hohen Anzahl von Nukleotiden, zwischen β5 und β7 mit n=20 und mit den gewählten Primern n=12. Außerdem wird mit dem einbezogenen Exon 1 der möglicherweise verfälschende Anteil der Genexpression hCG βlund β2 für die Gesamtexpression aller βhCG-Gene verhindert.

Bevorzugt werden zum Nachweis von Uteruskarzinomen einer Patientin Gewebe von Endometrium oder Zervix entnommen und die mRNA-Expression in dieser Gewebeprobe mit dem erfindungsgemäßen Verfahren analysiert.

Vorzugsweise werden die Werte der mRNA-Expression in Tumorgewebe mit den Werten der mRNA-Expression in gesundem Gewebe verglichen.

In einer besonders bevorzugten Variante der Verwendung wird dazu der Wert der mRNA-Expression von β7-hCG und/oder β6-hCG und/oder β6e hCG durch die Summe der Expression mRNA-Expression von Gesamt-βhCG geteilt und aus der Höhe des so erhaltenen Quotienten Rückschlüsse auf den Grad der Bösartigkeit des Tumors getroffen.

Es wurde gefunden, dass in einigen neoplastischen und tumorösen nicht- trophoblastären Geweben verstärkt hCG β5, β8, β3 und im neoplastischen Trophoblast zusätzlich hCG β7, β6 exprimiert werden.

Die Erfindung wird nachstehend in Ausführungsbeispielen näher erläutert, ohne auf diese beschränkt zu sein. Dabei zeigen :
Ausführungsbeispiel 1 : RT-PCR mit fluoreszenzmarkierten Primern zur Diagnostik der Rezeptivität der Uterusschleimhaut für eine Embryoimplantation
Ausführungsbeispiel 2 : RT-PCR mit nicht-markierten Primern zur Diagnostik der Rezeptivität der Uterusschleimhaut für eine Embryoimplantation
Ausführungsbeispiel 3 : RT-PCR mit nicht-markierten Primern zur retrospektiven Diagnostik der Rezeptivität der Uterusschleimhaut für eine Embryoimplantation

Die Veröffentlichung beschreibt weitere Beispiele anderer Anwendungen, worin zeigen:
Ausführungsbeispiel 4 : RT-PCR mit nicht-markierten Primern zur zur Tumordiagnostik
Ausführungsbeispiel S : RT-PCR mit fluoreszenzmarkierten Primern zur Tumordiagnostik
Ausführungsbeispiel 6 : RT-PCR mit fluoreszenzmarkierten Primern zur Tumordiagnostik

### Ausführungsbeispiel 1:

Zur Diagnostik der Rezeptivität der Uterusschleimhaut werden der Patientin Zellen mit einem Minikatheter aus der Gebärmutterhöhle oder mit einem Wattebausch aus der Zervix oder mit einem Holzspatel von der Mundschleimhaut entnommen. Die Zellen werden bis zur Weiterverarbeitung sofort bei minus 80° C eingefroren und gelagert. Zur Analyse wird aus den aufgenommenen Zellen eine Trizol-RNA-Extraktion durchgeführt, die cDNA des endometrialen hCG im nachfolgenden RT-PCR-Prozeß spezifisch amplifiziert und quantitativ erfaßt.

Es kann davon ausgegangen werden, dass die Anwesenheit von hCG ß7, ß6 und ß6e ein Indikator für eine optimale Implantation darstellt. Das Fehlen von hCG ß7, ß6 und ß6e zeigt das Gegenteil an: eine mögliche Implantation ist in diesem Zyklus nicht zu erwarten. Von besonderer Bedeutung ist der Fakt, dass mit der ßhCG-Diagnostik fehlendes oder hochaufgebautes sekretorisches Endometrium erkannt werden kann, so dass die Diagnose auch einen Therapiehinweis gibt. Zu beachten ist, dass hCG ß6 und ß6e im wesentlichen durch hCG ß7 repräsentiert werden kann (sechs Nukleotiddifferenzen im Exon 1 zu 24 Nukleotiddifferenzen zwischen ß7 und ß5). Mit der im Sequenzprotokoll angegebenen Auswahl verschiedener Primer können die hCG ß7, ß6, ß6e-Anteile summarisch, aber auch für hCG ß7 und hCG ß6 direkt bestimmt werden. Andererseits kann der Nachweis von geringem bis erhöhtem hCG ß5, ß8, und ß3 im endometrialen Gewebe oder deren Zellen einen Hinweis auf eine Tumorerkrankung darstellen. Die Gewebeproben können auch analog nach der Methode der fraktionierten Abrasio gewonnen werden.

Endometriales Gewebe (10 - 30 mg) oder Zellen dieser Herkunft werden sofort nach Entnahme in Flüssigstickstoff oder bei -80° C eingefroren. Für die Untersuchung der drei exprimierten Anteile hCG ß7, ß6 und ß6e sowie hCG ß8, ß5, ß3 und Gesagt-ßhCG wird die Total-RNA mit Trizol extrahiert und etwa 1 µg der RNA für 60 min bei 42° C unter Standardbedingungen und Einsatz von Oligo-dT(15)-Primer reversetranskribiert.

In diesem Ausführungsbeispiel wird zur Diagnostik der Rezeptivität der Uterusschleimhaut der Anteil der genspezifisch exprimierten βhCG-Amplifikate β7, β6, β6e im Endometrium zum Gesamt-hCG-Anteil von hCG β7, β6 plus hCG β5, β8, β3

bewertet. Dazu wird die nested RT-PCR-Methode benutzt, die im ersten RT-PCR-Schritt den Gesamtanteil von βhCG mit spezifischen Primern und dem Fluoreszenzmarker 1 und im folgenden nested PCR-Schritt mit diesem Produkt einmal hCG β7, β6, β6e mit Fluoreszenzmarker 2 und hCG β5, β8, β3 mit Fluoreszenzmarker 3 vermißt. Ein Software-Programm berechnet als Quotient den Anteil von hCG β7, β6, β6e zum Gesamtanteil des βhCG.

*Nutzung von Methoden:* Gewebeentnahme zur Diagnostik, Lagerung in Flüssigstickstoff, RNA-Extraktion (23), RT-PCR mit fluoreszenzmarkiertem Primerpaar, Erfassung der Gesamt-ßhCG-Expression ß5, ß8, ß3 und ß7, ß6 und ß6e über Exon1, Exon 2 und Exon 3, nested PCR-Methode mit unterschiedlich fluoreszenzmarkierten Primern jeweils für den ß7, ß6, ß6e- und eventuell ß5, ß8, ß3-Anteil; quantitative Auswertung als Quotient von ß7, ß6, ß6e-Fluoreszenzanteil zum Gesamt-hCG-Anteil ß7, ß6- plus ß5, ß8, ß3-Anteil für die Bewertung des hochaufgebauten sekretorischen endometrialen Gewebes, Ergebnis 1 bei Normalgewebe und Ergebnis > 0 bis 1 unterwertigem oder fehlenden sekretorisch trandformierten Gewebes im Ausführungsbeispiel 1; oder aber die absolute quantitative Auswertung der exprimierten Kopienzahlen für die genspezifischen ßhCG-Amplifikate ß7, ß6, ß6e und Gesamt-ßhCG nach Real time-RT-PCR im Vergleich zu ßhCG-sequenz-spezifischen Kalibratoren bei nicht-fluoreszenzmarkierten Primern und unter Verwendung von Standardmethoden für die Bewertung des normalen und neoblastischen Gewebes wie im Ausführungsbeispiel 2.

*Nutzung von Geräten und Material:* Gewebe in Flüssigstickstoff, Ultra Turrax-Gewebehomogenisation, Trizol-RNA-Extraktion, RT-PCR am Thermocycler, Fluoreszenzmessung des cDNA-Amplifikates am DNA Sequencer ABI 373A, Software Genescan 672 Fragment Analysis zur Auswertung, Flüssigstickstoff, Trizol, cDNA-Synthese-Kit, PCR-Amplifikationskit, ßhCG-Primer für Gesamt-ßhCG-Amplifikation und nested PCR für ß7, ß6, ß6e und ß5, ß8, ß3 zum Teil fluoreszenzmarkiert.

*Beschreibung der Methode für Ausführungsbeispiel 1:* Extraktion der Gesamt-RNA: Das frische Gewebematerial wird sofort nach der Entnahme in Flüssigstickstoff eingefroren. Die Gesamt-RNA wird mit der Methode nach Chomczynski und Sacchi (24) extrahiert, die gewonnene RNA spektrophotometrisch bei 260 nm / 280 nm quantifiziert, sofort weiterbearbeitet oder bei - 80° C gelagert.

*Reverse-Transkription:* 1 µg Gesamt-RNA wird in einem Reaktionsmix mit dem Totalvolumen von 5 µl nach der Standardmethode transkribiert: 10 mM Tris-HCl, pH 8,3, 50 mM KCl, 5 mM MgCl₂, 1mM jedes dNTP (dATP, dTTP, dCTP, dGTP), 200 ng Oligo dT-Primer pdT15, 12,5 U RNAse Inhibitor, 2,5 U AMV-Revertase. Inkubation des Reaktionsgemisches für 10 min bei 25 °C (Hybridisierung des Primers), 30 min bei 42°C (Reversetranskription) und 5 min bei 95 °C (Denaturierung der Revertase und des RNAse-Inhibitors) sowie Abkühlen auf 4 °C.

*PCR-Amplifikation der gesamten* ß*hCG-Transkripte:* Zum cDNA-Produkt wird im selben Tube der PCR-Mix von 20 µl im Gesamtvolumen von 25 µl für die Amplifikation des Gesamt-ßhCG-Transkriptes zugegeben: Endkonzentration von 10 mM Tris-HCl mit pH 8,3, 50 mM KCl, 1,5 mM MgCl₂, 200 µM dNTP, je 5 pmol der beiden gewählten Primer und 2,5 U Taq-DNA-Polymerase. Die Amplifikationsbedingungen sind nach vorheriger 3 min-Inkubation bei 95 °C dann 30 sec 95 °C, 30 sec 60 °C, 60 sec 72 °C für 35 Zyklen mit abschließenden 7 min bei 72 °C und schnellem Abkühlen auf 4 °C.

*Nested PCR für* ß*hCG* ß*7,* ß*6- und* ß*5,* ß*8,* ß*3-Transkripte:* 2 µl des 1:10.000 verdünnten PCR-Produktes werden zu einem Gesamtvolumen von 20 µl in ein PCR-Mix mit dem Endvolumen von 10 mM Tris-HCl, pH 8,3, 10 mM KCl, 3 mM MgCl₂, 50 µM dNTP, 0,1pmol Primer 2, 0,1 pmol Primer 3, 0,1 pmol des Primers 4 und 2 U Taq DNA Polymerase (Stoffel-Fragment) zugefügt. Die Reaktion wird über 5 Zyklen am Thermocycler für je 30 sec bei 95 °C und 30 sec bei 65 °C durchgeführt. Die nested PCR Reaktion wird auch mit Taq DNA Polymerase unter Standardbedingungen durchgeführt.

Das erhaltene Produkt enthält die zwei Amplifikationsprodukte für ßhCG ß7, ß6, ß6e und eventuell von hCG ß5, ß8, ß3 mit je einem differenten Fluoreszenzmarker für Primer 4 und Primer 3, und beide Transkripte enthalten zusätzlich einen dritten gemeinsamen Fluoreszenzmarker des Primers 2.

Für die Analyse am DNA Sequenzer (Perkin-Elmer Modell 373A oder vergleichbare Modelle) werden 2,5 µl des Produktes mit 2 µl Loading buffer und 0,5 µl Genescan Size Marker und der Elpho bei 8 % Acrylamid, 6 M Harnstoff und TBE-Puffer für 1 Stunde unterzogen. Die Ergebnisse werden mit der GeneScan 672 Software (Perkin-Elmer) analysiert unter Verwendung der ermittelten Fluoreszenzen für Gesamt-ßhCG-Transkripte und den ß7, ß6, ß6e- sowie eventuell den ß5, ß8, ß3-Fragmenten.

Der Transkriptionsindex wird, wie bei Bellet et al. (17) beschrieben nach dieser Methode errechnet.

### Ausführungsbeispiel 2

In diesem Ausführungsbeispiel wird zur Diagnostik der Rezeptivität der Uterusschleimhaut die absolute quantitative Auswertung der exprimierten Kopienzahlen für die genspezifischen ßhCG-Amplifikate ß7, ß6, ß6e und eventuell ßhCG ß5, ß8, ß3 nach Real time-RT-PCR im Vergleich zu ßhCG-spezifischen Kalibratoren bei nichtfluoreszenzmarkierten ßhCG-Primern für die Bewertung des normalen hochaufgebauten oder unterwertigen oder fehlenden sekretorisch transformierten endemetrialen Gewebes dargestellt.

Zur quantitativen Bestimmung der drei obengenannten ßhCG-Expressionsanteile wird die Real time-PCR am Light Cycler (Roche) oder vergleichbaren Geräten anderer Firmen wie Applied Biosystems für die Amplifikation der Tumor-cDNA eingesetzt. Für die Synthese der RNA- Standards der drei ßhCG-Expressionsanteile ß7, ß6, ß6e sowie eventuell ß5, ß8, ß3 und das Gesamt-ßhCG werden die drei zugehörigen Kalibrationsfragmente unter Standard-PCR-Bedingungen aus endo-metrialer, plazentarer und Tumor-cDNA amplifiziert. Dafür werden wieder die drei genannten, jetzt unmarkierten ßhCG Typ II (β8, ß5, ß3) -, ßhCG Typ I (ß7, ß6) - und Gesamt-ßhCG-spezifischen forward-hCG-Primer (Primer 1, 3, 4 oder andere) mit dem gemeinsamen reverse-ßhCG-Primer (Primer 2 oder andere) benutzt. Die erhaltenen PCR-Produkte werden im Plasmid-Vector pGEM-T geklont. Unter Verwendung der T7- und Sp6-Promotoren der pGEM-T-Vectors dient das Plasmid als Template für die in vitro-Bildung von RNA entsprechend des Herstellerprotokolls. Die gebildeten Standard-RNA werden gereinigt und seine Konzentration vermessen.

Die Real time-PCR-Amplifikation am Light-Cycler (Roche) oder ABI-Systemen (Applied Biosystems) bestimmt die Anzahl der gebildeten Genkopien für die zwei genspezifischen ßhCG-Expressionsgruppen Typ II (β8, ß5, ß3) und Typ I (ß7, ß6) sowie Gesamt-ßhCG im endometrialen Gewebe und in den RNA-Standards und unter Verwendung von den Primern 8, 9, 10 gegen 2 können auch die Einzelanteile von ß5, ß6 und ß7 erfaßt und absolut quantifiziert werden. Die PCR-Reaktion erfolgt im 20 µl-Reaktionsvolumen in den Endkonzentrationen von 1 x PCR-Puffer von 50 mM Tris-HCl (pH 8,3), 200 µM dNTPs, mit 0,5 µM der jeweils spezifischen forward- und reverse-ßhCG-Primer, 4 bis 5 mM MgCl₂, 0,5 U Taq Polymerase, SYBR Green I mit 1:3000 der Stammlösung (Molecular Probes) und 1 µl der Templates (Endometrium-cDNA gegen Standards bekannter Konzentration). Andere Methoden der Real time RT-PCR (TaqMan, FRET, Beacon) werden alternativ eingesetzt.

### Ausführungsbeispiel 3:

Zur retrospektiven Diagnostik der Rezeptivität der Uterusschleimhaut für eine Embryoimplantation wird der Patientin Menstrualblut entnommen und die korpuskulären Zellanteile werden abzentrifugiert. Die Zellen werden bis zur Weiterverarbeitung sofort bei minus 80° C eingefroren und gelagert. Zur Analyse der mRNA-Expression des endometrialen ßhCG wird wie in Ausführungsbeispiel 1 beschrieben verfahren.

Während für die prospektive Implantationsdiagnostik in der frühen bis mittleren Sekretionsphase des aktuellen Zyklus Gewebeproben des Endometriums, der Endocervix, Mundschleimhaut oder aus anderem ausgewählten Epithelium untersucht werden, um über die Qualität der sekretorischen Transformation und der zu erwartenden Rezeptivität des Endometriums (z. B. für die Entscheidung eines Embryotransfers oder Insemination im hormonell stimulierten Zyklus) zu entscheiden (zu befinden),
stellt die retrospektive Diagnostik der Rezeptivität der Uterusschleimhat (z. B. Menstrualblut als nicht-invasive Methode) nach erfolgtem Embryotransfer oder nur nach stimuliertem oder unstimulierten Zyklus eine wichtige und einfache Methode dar, Aussagen über die sekretorische Transformation des Endometriums des vorangegangen Zyklus als Diagnostik und/oder ggf. Therapiekontrolle und Aussage für den Folgezyklus zu treffen. Evt. kann diese Methode die übliche (klinisch genutzte) invasive Methode der Strichabrasio in ihrer Aussage ergänzen oder ersetzen.

### Ausführungsbeispiel 4:

Zur Tumordiagnostik werden der Patientin Zellen mit einem Minikatheter aus der Gebärmutterhöhle oder mit einem Wattebausch aus der Zervix oder mit einem Holzspatel von der Mundschleimhaut entnommen. Die Zellen werden bis zur Weiterverarbeitung sofort bei minus 80° C eingefroren und gelagert. Zur Analyse wird aus den aufgenommenen Zellen eine Trizol-RNA-Extraktion durchgeführt, die cDNA des endometrialen ßhCG im nachfolgenden RT-PCR-Prozeß spezifisch amplifiziert und quantitativ erfaßt.

Es kann davon ausgegangen werden, dass die Anwesenheit von hCG ß5, ß8 und ß6e ein Indikator für eine Tumorerkrankung darstellt. Das Vorhandensein von hCG ß7, ß6 und ß3 zeigt das Gegenteil an: eine mögliche nicht-trophoblastäre Tumorerkrankung kann ausgeschlossen werden. Von besonderer Bedeutung ist der Fakt, dass mit der ßhCG-Diagnostik aggressive Tumore erkannt werden kann, so dass die Diagnose auch einen Therapiehinweis gibt. Zu beachten ist, dass hCG ß6 und ß6e im wesentlichen durch hCG ß7 repräsentiert wird (siehe Ausführungsbeispiel 1). Die Untersuchungen werden vorteilhaft im Endometrium durchgeführt, um hier Karzinome zu erkennen. Gewebeproben können auch analog nach der Methode der fraktionierten Abrasio gewonnen werden.

Endometriales Gewebe oder Zellen dieser Herkunft (10 - 100 mg) werden sofort nach Entnahme in Flüssigstickstoff oder bei -80° C eingefroren. Für die Untersuchung der drei exprimierten Anteile hCG ß7, ß6 und ß6e sowie hCG ß8, 55, ß3 und Gesamt-ßhCG wird die Total-RNA mit Trizol extrahiert und etwa 1 µg der RNA für 60 min bei 42° C unter Standardbedingungen und Einsatz von Oligo-dT(15)-Primer reverse-transkribiert. *Nutzung von Methoden:* Gewebeentnahme zur Diagnostik, Lagerung in Flüssigstickstoff, RNA-Extraktion (23), RT-PCR mit fluoreszenzmarkiertem Primerpaar, Erfassung der Gesamt-ßhCG-Expression ß5, ß8, ß3 und ß7, ß6 und ß6e über Exon1, Exon 2 und Exon 3, nested PCR-Methode mit unterschiedlich fluoreszenzmarkierten Primern jeweils für den ß7, ß6, ß6e und eventuell ß5, ß8, ß3 Anteil; quantitative Auswertung als Quotient von ß7, ß6, ß6e-Fluoreszenzanteil zum

Gesamt-hCG-Anteil ß7, ß6- plus ß5, ß8, ß3-Anteil für die Bewertung des hochaufgebauten sekretorischen endometrialen Gewebes, Ergebnis 1 bei Normalgewebe und Ergebnis > 0 bis 1 unterwertigem oder fehlenden sekretorisch trandformierten Gewebes im Ausführungsbeispiel 4; oder aber die absolute quantitative Auswertung der exprimierten Kopienzahlen für die genspezifischen ßhCG-Amplifikate ß7, ß6, ß6e und Gesamt-ßhCG nach Real time-RT-PCR im Vergleich zu ßhCG-sequenz-spezifischen Kalibratoren bei nicht-fluoreszenzmarkierten Primern und unter Verwendung von Standardmethoden für die Bewertung des normalen und dardmethoden für die Bewertung des normalen und neoplastischen Gewebes wie im Ausführungsbeispiel 5.

*Nutzung von Geräten und Material:* Gewebe in Flüssigstickstoff, Ultra Turrax-Gewebehomogenisation, Trizol-RNA-Extraktion, RT-PCR am Thermocycler, Fluoreszenzmessung des cDNA-Amplifikates am DNA Sequencer ABI 373A oder vergleichbare Modelle, Software Genescan 672 Fragment Analysis zur Auswertung, Flüssigstickstoff, Trizol, cDNA-Synthese-Kit, PCR-Amplifikationskit, ßhCG-Primer für Gesamt-ßhCG-Amplifikation und nested RT-PCR für ß7, ß6, ß6e und ß5, ß8, ß3 zum Teil fluoreszenzmarkiert.

*Beschreibung der Methode für Ausführungsbeispiel 4:* Extraktion der Gesamt-RNA: Das frische Gewebematerial wird sofort nach der Entnahme in Flüssigstickstoff eingefroren. Die Gesamt-RNA wird mit der Methode nach Chomczynski und Sacchi (24) extrahiert, die gewonnene RNA spektrophotometrisch bei 260 nm / 280 nm quantifiziert, sofort weiterbearbeitet oder bei - 80° C gelagert.

*Reverse-Transkription:* 1 µg Gesamt-RNA wird in einem Reaktionsmix mit dem Totalvolumen von 5 µl nach der Standardmethode transkribiert: 10 mM Tris-HCl, pH 8,3, 50 mM KCl, 5 mM MgCl₂, 1mM jedes dNTP (dATP, dTTP, dCTP, dGTP), 200 ng Oligo dT-Primer pdT15, 12,5 U RNAse Inhibitor, 2,5 U AMV-Revertase. Inkubation des Reaktionsgemisches für 10 min bei 25 °C (Hybridisierung des Primers), 30 min bei 42 °C (Reversetranskription) und 5 min bei 95 °C (Denaturierung der Revertase und des RNAse-Inhibitors) sowie Abkühlen auf 4 °C.

*PCR Amplifikation der gesamten* ß*hCG-Transkripte:* Zum cDNA-Produkt wird im selben Tube der PCR-Mix von 20 µl im Gesamtvolumen von 25 µl für die Amplifikation des Gesamt-ßhCG-Transkriptes zugegeben: Endkonzentration von 10 mM Tris-HCl mit pH 8,3, 50 mM KCl, 1,5 mM MgCl₂, 200 µM dNTP, 5 pmol Primer 1, 5 pmol Primer 2 und 2,5 U Taq-DNA-Polymerase. Die Amplifikationsbedingungen sind nach vorheriger 3 minInkubation bei 95 °C dann 30 sec 95 °C, 30 sec 60 °C, 60 sec 72 °C für 35 Zyklen mit abschließenden 7 min bei 72 °C und schnellem Abkühlen auf 4 °C.

*Nested PCR für* ß*hCG* ß*7,* ß*6- und* ß*5,* ß*8,* ß*3-Transkripte:* 2 µl des 1:10.000 verdünnten PCR-Produktes werden zu einem Gesamtvolumen von 20 µl in ein PCR-Mix mit dem Endvolumen von 10 mM Tris-HCl, pH 8,3, 10 mM KCl, 3 mM MgCl₂, 50 µM dNTP, 0,1pmol Primer 2, 0,1 pmol Primer 3, 0,1 pmol des Primers 4 und 2 U Taq DNA Polymerase (Stoffel-Fragment) zugefügt. Die Reaktion wird über 5 Zyklen am Thermocycler für je 30 sec bei 95 °C und 30 sec bei 65 °C durchgeführt. Die nested PCR-Reaktion wird auch mit Taq DNA Polymerase unter Standardbedingungen durchgeführt.

Das erhaltene Produkt enthält die zwei Amplifikationsprodukte für ßhCG ß7, ß6, ß6e und von hCG ß5, ß8, ß3 mit je einem differenten Fluoreszenzmarker für Primer 4 und Primer 3, und beide Transkripte enthalten zusätzlich einen dritten gemeinsamen Fluoreszenzmarker des Primers 2.

Für die Analyse am DNA Sequenzer (Perkin-Elmerm Modell 373A oder vergleichbare Modelle) werden 2,5 µl des Produktes mit 2 µl Loading buffer und 0,5 µl Genescan Size Marker und der Elpho bei 8 % Acrylamid, 6 M Harnstoff und TBE-Puffer für 1 Stunde unterzogen. Die Ergebnisse werden mit der GeneScan 672 Software (Perkin-Elmer) analysiert unter Verwendung der ermittelten Fluoreszenzen für Gesamt-ßhCG-Transkripte und den ß7, ß6, ß6e- sowie eventuell den ß5, ß8, ß3-Fragmenten.

Der Transkriptionsindex wird, wie bei Bellet et al. (17) beschrieben nach dieser Methode errechnet.

### Ausführungsbeispiel 5

Tumorgewebe (50 - 200 mg) wird sofort nach Entnahme in Flüssigstickstoff eingefroren. Für die Untersuchung der drei exprimierten Anteile hCG ß8, ß5, 33 und hCG ß7, ß6 sowie Gesamt-ßhCG wird die Total-RNA mit Trizol extrahiert und etwa 1 µg der RNA für 60 min bei 42° C unter Standardbedingungen und Einsatz von Oligo- dT(15) -Primer reverse-transkribiert.

Zur quantitativen Bestimmung der drei obengenannten ßhCG-Expressionsanteile wird die Real time-PCR am Light Cycler (Roche) oder vergleichbaren Geräten anderer Firmen wie Applied Biosystems für die Amplifikation der Tumor-cDNA eingesetzt. Für die Synthese der RNA-Standards der drei ßhCG-Expressionsanteile ß8, ß5, ß3 sowie ß7, ß6 und Gesamt-ßhCG werden die drei zugehörigen Kalibrationsfragmente unter Standard-PCR-Bedingungen aus endometrialer, plazentarer und Tumor-cDNA amplifiziert. Dafür werden wieder die drei genannten, jetzt unmarkierten ßhCG Typ II (β8, ß5, ß3)-, ßhCG Typ I (ß7, ß6) - und Gesamt-ßhCG-spezifischen forward-ßhCG-Primer (Primer 1, 3 und 4 oder andere) mit dem gemeinsamen reverse-ßhCG-Primer (Primer 2 oder andere) benutzt. Die erhaltenen PCR-Produkte werden im Plasmid-Vector pGEM-T geklont. Unter Verwendung der T7- und Sp6-Promotoren der pGEM-T-Vectors dient das Plasmid als Template für die in vitro-Bildung von RNA entsprechend des Herstellerprotokolls. Die gebildeten Standard-RNA werden gereinigt und seine Konzentration vermessen.

Die Real time-PCR-Amplifikation am Light Cycler (Roche) oder ABI-Systemen (Applied Biosystems) bestimmt die Anzahl gebildeter Genkopien für die zwei genspezifischen ßhCG-Expressionsgruppen Typ II (β8, ß5, ß3) und Typ I (ß7, ß6) sowie Gesamt-ßhCG im Tumorgewebe und in den RNA-Standards und unter Verwendung von den Primern 8, 9, 10 gegen 2 können auch die Einzelanteile von hCG ß5, ß6 und ß7 erfaßt und absolutquantifiziert werden. Die PCR-Reaktion erfolgt im 20 µl-Reaktionsvolumen in den Endkonzentrationen von 1 x PCR-Puffer von 50 mM Tris-HCl (pH 8,3), 200 µM dNTPs, mit 0,5 µM der jeweils spezifischen forward- und reverse-ßhCG-Primer, 4 bis 5 mM MgCl₂, 0,5 U Taq Polymerase, SYBR Green I mit 1:3000 der Stammlösung (Molecular Probes) und 1 µl der Templates (Tumor-cDNA oder Standards bekannter Konzentration). Andere Methoden der Real time RT-PCR (TaqMan, FRET, Beacon) werden ebenso eingesetzt.

Die Erfindung beansprucht auch die Real time-Messung als one tube-RT-PCR oder die Verwendung anderer Methoden zur quantitativen Erfassung der Expression spezifischer Genkopien neben SYBR Green I, wie zum Beipiel der Einsatz von genspezifischen Oligonukleotiden als Hybridisierungsproben mit unterschiedlichen Farbstoff- oder Fluoreszenzmarker-Anbindung (TaqMan, FRET, Beacon).

### Ausführungsbeispiel 6

Tumorgewebe wird zur Diagnostik entnommen und in Flüssig-Stickstoff gelagert. Nach einer RNA-Extraktion (23) folgt eine RT-PCR mit fluoreszenzmarkiertem Primerpaar entsprechend Ausführungsbeispiel 4. Die Gesamt-ßhCG-Expression ß5, ß8, ß3 und ß7, ß6 über Exon1, Exon 2 und Exon 3 wird in einer nested PCR mit fluoreszenzmarkierten Primern jeweils für den ß7, ß6- und ß5, ß8, ß3-Anteil erfasst und als Quotient von ß5, ß8, ß3-Anteil zu ß7, ß6- plus ß5, ß8, ß3-Anteil für die Bewertung des neoplastischen und tumorösen nicht-trophoblastären Gewebes wie folgt ausgewertet:
Ergebnis 0 bei Normalgewebe und Ergebnis > 0 bis 1 bei neoplastischem Gewebe - entsprechend Ausführungsbeispiel 4 Es erfolgt eine absolute quantitative Auswertung der exprimierten Kopienzahlen für die genspezifischen ßhCG-Amplifikateß5, ß8, ß3 sowie ß7, ß6 und Gesamt-ßhCG nach Real time-RT-PCR im Vergleich zu hCGsequenzspezifischen Kalibratoren für die Bewertung des normalen und neoplastischen Gewebes im Ausführungsbeispiel 5.

Nutzung von Geräten und Material: Gewebe in Flüssigstickstoff, Ultra Turrax-Gewebehomogenisation, Trizol-RNA-Extraktion, RT-PCR am Thermocycler, Fluoreszenzmessung des cDNA-Amplifikates am DNA Sequencer ABI 373A, Software Genescan 672 Fragment Analysis zur Auswertung, Flüssigstickstoff, Trizol, cDNA-Synthese-Kit, PCR-Amplifikationskit, ßhCG-Primer für Gesamt-ßhCG-Amplifikation und nested PCR für ß5, ß8, ß3 und ß7, ß6, zum Teil fluoreszenzmarkiert.

### Beschreibung der Methode für Ausführungsbeispiel 6:

Extraktion der Gesamt-RNA: Das Gewebematerial wird sofort nach Entnahme in Flüssigstickstoff eingefroren. Die Gesamt-RNA wird mit der Methode nach Chomczynski und Sacchi (24) extrahiert, die gewonnene RNA spektrophotometrisch bei 260 nm / 280 nm quantifiziert, sofort weiterbearbeitet oder bei - 80° C gelagert.

*Reverse-Transkription:* 1 µg Gesamt-RNA wird in einem Reaktionsmix mit dem Gesamtvolumen von 5 µl nach der Standardmethode transkribiert: 10 mM Tris-HCl, pH 8,3, 50 mM KCl, 5 mM MgCl₂, 1mM jedes dNTP (dATP, dTTP, dCTP, dGTP), 200 ng Oligo dT-Primer pdT15, 12,5 U RNAse Inhibitor, 2,5 U AMV-Revertase (Roche). Inkubation des Reaktionsgemisches für 10 min bei 25 °C (Hybridisierung des Primers), 30 min bei 42 °C (Reversetranskription) und 5 min bei 95 °C (Denaturierung der Revertase und des RNAse-Inhibitors) sowie Abkühlen auf 4 °C.

PCR-Amplifikation der gesamten ßhCG-Transkripte: Zum cDNA-Produkt wird im selben Tube der PCR-Mix von 20 µl im Gesamtvolumen von 25 µl für die Amplifikation des Gesamt-ßhCG-Transkriptes zugegeben: Endkonzentration von 10 mM Tris-HCl, pH 8,3, 50 mM KCl, 1,5 mM MgCl₂, 200 µM dNTP, je 5 pmol der beiden gewählten Primer und 2,5 U Taq-DNA-Polymerase. Die Amplifikations-bedingungen sind nach vorheriger 3 min-Inkubation bei 95 °C dann 30 sec 95 °C, 30 sec 60 °C, 60 sec 72 °C für 35 Zyklen mit abschließenden 7 min bei 72 °C und schnellem Abkühlen auf 4 °C.

*Nested PCR für* ß*hCG* ß*7,* ß*6- und* ß*5,* ß*8,* ß*3-Transkripte:* 2 µl des 1:10.000 verdünnten PCR-Produktes werden zu einem Gesamtvolumen von 20 µl in ein PCR-Mix mit dem Endvolumen von 10 mM Tris-HCl, pH 8,3, 10 mM KCl, 3 mM MgCl₂, 50 µM dNTP, 0,1 pmol Primer 2, 0,1 pmol Primer 3, 0,1 pmol des Primers 4 und 2 U Taq DNA Polymerase (Stoffel-Fragment) zugefügt. Die Reaktion wird über 5 Zyklen am Thermocycler für je 30 sec bei 95 °C und 30 sec bei 65 °C durchgeführt. Die nested PCR Reaktion wird auch mit Taq DNA Polymerase unter Standardbedingungen durchgeführt.

Das erhaltene Produkt enthält die zwei Amplifikationsprodukte für ßhCG ß5, ß8, ß3 und ß7, ß6 mit je einem differenten Fluoreszenzmarker für Primer 3 und Primer 4, und beide Transkripte enthalten zusätzlich einen dritten gemeinsamen Fluoreszenzmarker des Primers 2.

Für die Analyse am DNA Sequenzer (Perkin-Elmer-Modell 373A oder vergleichbare Modelle) werden 2,5 µl des Produktes mit 2 µl Loading buffer und 0,5 µl Genescan Size Marker und der Elpho bei 8 % Acrylamid, 6 M Harnstoff und TBE-Puffer für 1 Stunde unterzogen. Die Ergebnisse werden mit der GeneScan 672 Software (Perkin-Elmer) analysiert unter Verwendung der ermittelten Fluoreszenzen für Gesamt-ßhCG-Transkripte und den ß7, ß6- sowie ß5, ß8, ß3-Fragmenten.

Der Transkriptionsindex wird, wie bei Bellet et al. (17) beschrieben, nach dieser Methode als Quotient von ßhCG ß7, ß6 zur Summe von ßhCG ß7, ß6 und ß5, 8, ß3 errechnet.

Die vorgestellte Erfindung bringt eine Reihe wesentlicher Vorteile mit sich. Die erhaltenen Ergebnisse gewinnen an Zuverlässigkeit, weil es mehrere Ansatzpunkte für den Indikator gibt. Im Unterschied zu der bekannten technischen Lösung, die ausschließlich auf die Punktmutation 117 in Exon 3 abstellt, bezieht unsere Lösung Exon 2 und ein Promotorgen ein. Unser Verfahren ermöglicht eine Unterscheidung in bösartige und in gutartige Tumore mit den gewünschten Folgen für einen Therapieansatz. Das basiert auf der Erkenntnis, dass der Grad der Bösartigkeit eines nicht-trophoblastären Tumors durch die Anwesenheit von hCG β5, ß8, ß3 indiziert wird. Dessen Konzentration wird im Ausführungsbeispiel 4 als Fluoreszenzwert gemessen und zu hCG ß5, ß8, ß3 in Beziehung gesetzt, indem der Quotient von hCG ß5, ß8, ß3 zur Summe von hCG ß5, ß8, ß3 plus hCG ß6, ß7 gebildet wird.

Im Ausführungsbeispiel 5 wird die Anwesenheit von hCG ß5, ß8, ß3 durch Real time-RT-PCR durch die Kopienzahl seiner Genexpression im Vergleich zur sequenzspezifischen ßhCG-Standardreihe wie auch von hCG ß7, ß6 absolut quantifiziert.

Das erfindungsgemäße Verfahren wird vorzugsweise mit einem Testkit durchgeführt, der die folgenden Bestandteile enthält:

| | **Reaktionslösungen** | **Ingredenzien** |
|---|---|---|
| 1. | Primer 1 | nicht-markierter Primer 1 |
| 2. | Primer 2 | fluoreszenzmarkierter Primer 2 |
| 3. | Primer 3 | fluoreszenzmarkierter Primer 3 |
| 4. | Primer 4 | fluoreszenzmarkierter Primer 4 |
| 5. | RT-Reaktionsmix | RT-Reaktionspuffer mit dNTPs, Oligo-pdT15, RNAse-Inhibitor für cDNA-Bildung |
| 6. | Reverse Transkriptase | Stammlösung für RT |
| 7. | PCR-Reaktionsmix | PCR-Reaktionspuffer |
| 8. | PCR-Polymerase | Taq-DNA-Polymerase |
| 9. | nested PCR-Reaktionsmix | nested PCR-Reaktionspuffer |

Der mRNA-Quantifizierungskit für ßhCG gene ß5, ß7 gestattet die hochempfindliche und spezifische Bestimmung der Genexpression von ßhCG im normalen und Tumorgewebe für die Diagnostik und Therapiekontrolle.

Die mit den Methoden der Real time-RT-PCR amplifizierten spezifischen ßhCG ß5- und ßhCG ß7-Kopien können über einen breiten Meßbereich mit je einem Satz bereitstehender Kalibrationsstandards von ßhCG ß5- und ßhCG ß7-mRNA erfaßt werden.

### Abkürzungsverzeichnis

- cDNA: komplementäre DNA
- CTP: C-terminales Peptid
- ELISA: Enzyme linked immmunosorbent assay
- ET: Embryotransfer
- hCG: humanes Choriongonadotropin
- α-hCG: alpha Untereinheit des hCG
- β-hCG: beta Untereinheit des hCG
- thCG: trophoblastär exprimierte Form des hCG
- HRP: Horse radish peroxidase - Meerrettichperoxidase
- βLH: Luteinisierendes Hormon
- PBS: Phosphat buffer saline (Natriumphosphatpuffer)
- pdT15: Primer poly-deoxyThymidin aus 15 Monomeren aufgebaut 15
- Mab: monoklonaler Antikörper
- MEIA: Mikropartikel Enzymimmunoassay
- M: mol/Liter
- mRNA: Messenger-RNA
- PCR: Polymerasekettenreaktion
- RT-PCR: Reverse Transkriptase PCR

### Zitierte Nicht-Patentliteratur:

(1) J.C.Pierce, T.F.Parsons, Annu.Rev.Biochem., 50 (1981) 465-495
(2) P.A.Rothman et al. Mol.Reprod.Dev., 33 (1992) 1-6
(3) S.Dirnhofer et al. J.Clin.Endocrinol.Metab., 81 (1996) 4212-4217
(4) Z.M.Lei et al. , J.Clin.Endocrinol.Metab., 77 (1993) 863-972
(5) T.Yokotani et al. Int.J.Cancer, 71 (1997) 539-544
(6) P.Berger et al. FEBS Lett., 343 (1994) 229-233
(7) I.Marcilliac et al., Cancer Res., 52 (1992) 3901-3907
(8) H.Alfthan, et al., Clin.Chem., 38 (1992) 1981-1987
(9) V.Lazar et al., Cancer Res., 55 (1995) 3735-3738
(10) P.N.Span et al., J.Endocrinol., 172 (2002) 489-495
(11) M.Lundin, et al., Int.J.Cancer, 95 (2001) 18-22
(12) K.Hotakainen et al., Brit.J.Cancer, 86 (2001) 185-189
(13) D.S.Hoon, et al., Int.J.Cancer, 69 (1996) 369-374
(17) D.Bellet, et al., Cancer Res., 57 (1997) 516-523
(18) P.K.Hotakainen et al., Mol.Cell.Endocrinol., 162 (2000) 79-85
(19) A.K.Miller-Lindholm, et al., 138 (1997) 5459-5465
(20) S.Madersbacher, et al., Cancer Res., 54 (1994) 5096-5100
(21) R.Oyasu, et al., Arch.Pathol.Lab.Med., 119 (1994) 715-717

## Patentansprüche

1. Verfahren zur Bestimmung der Aufnahmebereitschaft der Uterusschleimhaut für die Implantation eines Embryos, bei dem RNA aus einer Blut- und/oder Gewebeprobe isoliert wird und in dieser Probe eine quantitative Messung der Expression oder Überexpression der mRNA von β7-hCG und/oder β6-hCG und/oder einer mRNA, deren cDNA eine Sequenz nach SEQ ID No 7 aufweist, erfolgt, wobei eine Anwesenheit von β7-hCG und/oder β6-hCG und/oder einer mRNA, deren cDNA eine Sequenz nach SEQ ID No. 7 aufweist, ein Indikator für die optimale Implantation darstellt.

2. Verfahren nach Anspruch 1, **dadurch gekennzeichnet, dass** zusätzlich eine quantitative Messung der Gesamt-βhCG-mRNA-Expression oder der mRNA-Expression von β5-hCG und/oder β8-hCG und/oder β3-hCG erfolgt und mit der mRNA-Expression von β7-hCG- und/oder β6-hCG und/oder einer mRNA, deren cDNA eine Sequenz nach SEQ ID No 7 aufweist, in Relation gesetzt wird.

3. Verfahren nach Anspruch 1 oder 2, **dadurch gekennzeichnet, dass** die quantitative Messung der mRNA-Expression mittels quantitativer RT-PCR oder Real-time-RT-PCR erfolgt.

4. Verfahren nach Anspruch 3, **dadurch gekennzeichnet, dass** basierend auf der durch die Reverse Transkriptase (RT) erhaltenen cDNA in einem ersten PCR-Schritt mit mindestens einem ersten Primerpaar Gesamt-βhCG-cDNA amplifiziert wird, wobei das erste Primerpaar sowohl mit cDNA von β5-hCG, β8-hCG, β3-hCG, als β7-hCG und β6-hCG und einer mRNA, deren cDNA eine Sequenz nach SEQ ID No 7 aufweist, hybridisiert, und in einem anschließenden zweiten PCR-Schritt mit mindestens einem dritten Primer die cDNA von β7-hCG- und/oder β6-hCG und/oder einer mRNA, deren cDNA eine Sequenz nach SEQ ID No. 7 aufweist, spezifisch amplifiziert wird, wobei der dritte Primer mit cDNA von β7-hCG und β6-hCG und einer mRNA, deren cDNA eine Sequenz nach SEQ ID No. 7 aufweist, spezifisch hybridisiert, jedoch nicht mit cDNA von β5-hCG, β8-hCG und β3-hCG.

5. Verfahren nach Anspruch 4, **dadurch gekennzeichnet, dass** in dem zweiten PCR-Schritt zusätzlich mit mindestens einem vierten Primer die cDNA von β5-hCG und/oder β8-hCG und/oder β3-hCG spezifisch amplifiziert wird, wobei der vierte Primer mit cDNA von β5-hCG, β8-hCG und β3-hCG spezifisch hybridisiert, jedoch nicht mit cDNA von β7-hCG und β6-hCG und einer mRNA, deren cDNA eine Sequenz nach SEQ ID No 7 aufweist.

6. Verfahren nach Anspruch 4 oder 5, **dadurch gekennzeichnet, dass** als erstes Primerpaar Oligonukleotidpaare aus der Gruppe von Sequenzen gemäß SEQ ID No 1 und SEQ ID No 2, gemäß SEQ ID No 1 und SEQ ID No 11 oder SEQ ID No 14 und SEQ ID No 2 im ersten PCR-Schritt und als dritter Primer ein Oligonukleotid aus der Gruppe von Sequenzen gemäß SEQ ID NO 3, SEQ ID No 9, SEQ ID No 10, SEQ ID No 13 und SEQ ID No 16 im zweiten PCR-Schritt eingesetzt werden.

7. Verfahren nach Anspruch 5 oder 6, **dadurch gekennzeichnet, dass** als vierter Primer ein Oligonukleotid aus der Gruppe von Sequenzen gemäß SEQ ID No 4, SEQ ID No 8, SEQ ID No 12 und SEQ ID No 15 im zweiten PCR-Schritt eingesetzt wird.

8. Verfahren nach einem der Ansprüche 4 bis 7, **dadurch gekennzeichnet, dass** mindestens ein Primer fluoreszenzmarkiert ist.

9. Verfahren nach Anspruch 8, **dadurch gekennzeichnet, dass** ein Primer des ersten Primerpaars, der dritte Primer und gegebenenfalls der vierte Primer mit Fluoreszenzmarkern die sich in Ihren Adsorptions- und/oder Emissionsspektren zueinander unterscheiden, versehen sind.

10. Verwendung des Verfahrens nach einem der Ansprüche 1 bis 9 zur prospektiven oder retrospektiven Diagnostik einer endometrialen Rezeptivität für eine Embryoimplantation.

11. Verwendung nach Anspruch 10, **dadurch gekennzeichnet, dass** einer Patientin Peripherblut oder Gewebe von Endometrium oder Zervix entnommen wird und die Analyse der mRNA-Expression in dieser Blut- oder Gewebeprobe erfolgt und aus der Höhe der ermittelten mRNA-Expression von β7-hCG und/oder β6-hCG und/oder einer mRNA, deren cDNA eine Sequenz nach SEQ ID No 7 aufweist, Rückschlüsse auf die Aufnahmebereitschaft der Gebärmutter für einen Embryo im aktuellen Zyklus getroffen werden.

12. Verwendung nach Anspruch 10, **dadurch gekennzeichnet, dass** die Analyse der mRNA-Expression in einer Probe von Menstrualblut einer Patientin erfolgt und aus der Höhe der ermittelten mRNA-Expression von β7-hCG und/oder β6-hCG und/oder einer mRNA, deren cDNA eine Sequenz nach SEQ ID No 7 aufweist, im abgelaufenen Zyklus Prognosen auf die potentielle Aufnahmebereitschaft der Gebärmutter für einen Embryo im Folgezyklus erstellt werden.

13. Verwendung eines Primers zur Bestimmung der Aufnahmebereitschaft der Uterusschleimhaut für die Implantation eines Embryos, wobei der Primer eine Primersequenz gemäß SEQ ID No 3 oder SEQ ID No 4 oder einer der SEQ ID No 8 bis SEQ ID No 16 aufweist.

14. Verwendung eines diagnostischen Kits zur Bestimmung der Aufnahmebereitschaft der Uterusschleimhaut für die Implantation eines Embryos durch quantitative RT-PCR, das diagnostische Kit enthaltend jeweils eine Menge
a.) Oligo-dT,
b.) des Enzyms Reverse Transkriptase,
c.) ein Primerpaar ausgewählt aus der Gruppe von Sequenzen gemäß SEQ ID No 1 und SEQ ID No 2, gemäß SEQ ID No 1 und SEQ ID No 11 oder SEQ ID No 14 und SEQ ID No 2,
d.) einer über 80°C beständigen DNA-Polymerase,
e.) Reaktionspuffer.

15. Verwendung des diagnostischen Kits nach Anspruch 14, **dadurch gekennzeichnet, dass** er eine Menge eines vierten Primers ausgewählt aus der Gruppe von Sequenzen gemäß SEQ ID No 4, SEQ ID No 8, SEQ ID No 12 und SEQ ID No 15 enthält.

16. Verwendung des diagnostischen Kits nach einem der Ansprüche 14 oder 15, **dadurch gekennzeichnet, dass** mindestens ein Primer fluoreszenzmarkiert ist.

17. Verwendung des diagnostischen Kits nach Anspruch 16, **dadurch gekennzeichnet, dass** ein Primer des ersten Primerpaars, der dritte Primer und gegebenenfalls der vierte Primer mit Fluoreszenzmarkern die sich in Ihren Adsorptions- und/oder Emissionsspektren zueinander unterscheiden, versehen sind.

18. Verwendung des diagnostischen Kits nach einem der Ansprüche 14 bis 17, **dadurch gekennzeichnet, dass** er eine definierte Menge mRNA oder cDNA von β5-hCG und/oder β7-hCG als Standard enthält.

19. Verwendung des diagnostischen Kits nach einem der Ansprüche 14 bis 18 zur prospektiven oder retrospektiven Diagnostik der Aufnahmebereitschaft der Uterusschleimhaut für die Implantation des Embryos.

20. Verwendung einer cDNA zur Bestimmung der Aufnahmebereitschaft der Uterusschleimhaut für die Implantation eines Embryos, wobei die cDNA eine Gensequenz, die unter SEQ ID No 7 fällt und/oder eine Gensequenz nach SEQ ID No 5 und/oder SEQ ID No 6 aufweist und/oder eine Gensequenz codierend für ein Protein mit der Aminosäuresequenz gemäß SEQ ID No 17 oder 18 als Marker zur prospektiven oder retrospektiven Diagnostik der Aufnahmebereitschaft der Uterusschleimhaut für die Implantation des Embryos.

## Claims

1. A method for determining receptiveness of an endometrium to implantation of an embryo, wherein RNA from a blood and/or tissue probe is isolated and wherein in this probe a quantitative measurement is made of expression or over-expression of mRNA of β7-hCG and/or of β6-hCG and/or of an mRNA, a cDNA of which comprises a sequence according to SEQ ID No 7, wherein a presence of β7-hCG and/or β6-hCG and/or an mRNA, of which a cDNA comprises a sequence according to SEQ ID No 7, is an indication for an optimised implantation.

2. The method of claim 1, **characterized in that**, in addition, a quantitative measurement is made of the total expression of βhCG-mRNA or of the expression of mRNA of β5-hCG and/or β8-hCG and/or β3-hCG which is put into relation with the expression of mRNA of β7-hCG and/or of β6-hCG and/or of a mRNA, a cDNA of which comprises a sequence according to SEQ ID No 7.

3. The method of claim 1 or 2, **characterized in that** the quantitative measurement of expression of mRNA is made by quantitative RT-PCR or Real-time-RT-PCR.

4. The method of claim 3, **characterized in that**, based on cDNA obtained by the reverse transcriptase (RT), in a first PCR-step the total βhCG-cDNA is amplified by use of a first pair or primers, wherein the first pair of primers hybridises with cDNA of β5-hCG, of β8-hCG, of β3-hCG, and of β7-hCG, of β6-hCG and of a mRNA, of which a cDNA comprises a sequence according to SEQ ID No 7, and in a subsequent second PCR-step cDNA of β7-hCG and/or β6-hCG and/or a mRNA, of which a cDNA comprises a sequence according to SEQ ID No 7, is specifically amplified by use of a third primer, wherein the third primer specifically hybridises with cDNA of β7-hCG and β6-hCG and a mRNA, of which a cDNA comprises a sequence according to SEQ ID No 7, but does not hybridize with cDNA of β5-hCG, β8-hCG or β3-hCG.

5. The method of claim 4, **characterized in that** the cDNA of β5-hCG and/or of β8-hCG and/or of β3-hCG is specifically amplified with a fourth primer in the second PCR-step, wherein the fourth primer specifically hybridises with cDNA of β5-hCG, β8-hCG and β3-hCG, but does not hybridize with cDNA of β7-hCG β6-hCG or a mRNA, a cDNA of which comprises a sequence according to SEQ ID No 7.

6. The method of claim 4 or 5, **characterized in that** pairs of oligonucleotides are used as the firsts pair of primers selected from the group of sequences according to SEQ ID No 1 and SEQ ID No 2, according to SEQ ID No 1 and SEQ ID No 11 or SEQ ID No 14 and SEQ ID No 2 in the first PCR-step and an oligonucleotide is used as the third primer that is selected from the group of sequences according to SEQ ID NO 3, SEQ ID No 9, SEQ ID No 10, SEQ ID No 13 and SEQ ID No 16 in the second PCR-step.

7. The method of claim 5 or 6, **characterized in that** an oligonucleotide selected from the group of sequences according SEQ ID No 4, SEQ ID No 8, SEQ ID No 12 and SEQ ID No 15 are used as the fourth primer in the second PCR-step.

8. The method of any one of claims 4 to 7, **characterized in that** at least one primer comprises a fluorescence marker.

9. The method of claim 8, **characterized in that** a primer of the first pair of primers, the third primer and optionally the fourth primer comprise fluorescence markers that differ in the absorption and/or emission spectra with respect to each other.

10. A use of the method of any one of claims 1 to 9 for a prospective or retrospective diagnosis of an endometrial receptivity for implantation of an embryo.

11. The use according to claim 10, **characterized in that** a sample of peripheral blood or of tissue of endometrium or cervix of a patient is taken and an the expression of mRNA in this sample of blood or tissue is analysed and a prognosis of the receptiveness of the uterus to an embryo in the actual cycle is made based on the level of the determined expression of mRNA of β7-hCG and/or β6-hCG and/or a mRNA, of which a cDNA comprises a sequence according to SEQ ID No 7.

12. The use according to claim 10, **characterized in that** the analysis of the expression of mRNA is made in a sample taken from menstrual blood of a patient and a prognosis of the receptiveness of the uterus to an embryo in the subsequent cycle is made based on the value of the determined expression of mRNA of β7-hCG and/or β6-hCG and/or a mRNA, of which a cDNA comprises a sequence according to SEQ ID No 7 in the last cycle.

13. The use of a primer for determination of receptiveness of endometrium to implantation of an embryo, wherein the primer comprises a primer sequence according to SEQ ID No 3 or SEQ ID No 4 or any one of SEQ ID No 8 to SEQ ID No 16.

14. The use of a diagnostic kit for determination of receptiveness of endometrium to implantation of an embryo by quantitative RT-PCR, wherein the diagnostic kit comprises and amount of
a.) Oligo-dT,
b.) the enzyme reverse transcriptase,
c.) a pair of primers selected from the group or sequences according to SEQ ID No 1 and SEQ ID No 2, according to SEQ ID No 1 and SEQ ID No 11 or SEQ ID No 14 and SEQ ID No 2,
d.) a DNA-polymerase that withstand more than 80°C,
e.) reaction buffer.

15. The use of the diagnostic kit of claim 14, **characterized in that** the diagnostic kit comprises an amount of a fourth primer selected from the group of sequences according to SEQ ID No 4, SEQ ID No 8, SEQ ID No 12 and SEQ ID No 15.

16. The use of the diagnostic kit of claim 14 or 15, **characterized in that** at least one primer comprises a fluorescence marker.

17. The use of the diagnostic kit of claim 15, **characterized in that** one primer of the first pair of primers, the third primer and optionally the fourth primer comprise fluorescence markers that differ in the absorption and/or emission spectra with respect to each other.

18. The use of the diagnostic kit of any one of claims 14 to 17, **characterized in that** the diagnostic kit comprises a defined amount of mRNA or cDNA of β5-hCG and/or β7-hCG as a standard.

19. The use of the diagnostic kit of any one of claims 14 to 18 for a prospective or retrospective diagnosis of an endometrial receptiveness to the implantation of the embryo.

20. A use of a cDNA for determination of receptiveness of endometrium to implantation of an embryo wherein the cDNA comprises a gene sequence according to SEQ ID No 7 and/or a gene sequence according to SEQ ID No 5 and/or SEQ ID No 6 and/or a gene sequence coding for a protein with an amino acid sequence according to SEQ ID No 17 or 18 as marker a prospective or retrospective diagnosis of the endometrial receptiveness to the implantation of the embryo.

## Revendications

1. Procédé de détermination de la réceptivité de la muqueuse utérine pour l'implantation d'un embryon, dans lequel un ARN est isolé à partir d'un échantillon de sang et/ou de tissu et dans lequel on effectue une mesure quantitative de l'expression ou de la surexpression du ARNm de β7 - hCG et/ou de β6 - hCG et/ou d'un ARNm dont l'ADNc présente une séquence selon SEQ ID N° 7, une présence de β7 - hCG et/ou de β6 - hCG et/ou d'un ARNm dont l'ADNc présente une séquence selon SEQ ID N° 7 représentant un indicateur pour l'implantation optimale.

2. Procédé selon la revendication 1, **caractérisé en ce que** l'on effectue en supplément une mesure quantitative de l'expression totale de β hCG - ARNm ou de l'expression de ARNm de β5 - hCG et/ou de β8 - hCG et/ou de β3 - hCG et on la met en relation avec l'expression de ARNm de β7 - hCG et/ou de β6 - hCG et/ou d'un ARNm dont l'ADNc présente une séquence selon SEQ ID N° 7.

3. Procédé selon la revendication 1 ou 2, **caractérisé en ce que** l'on effectue la mesure quantitative de l'expression de ARNm au moyen d'une RT - PCR ou d'une RT - PCR en temps réel.

4. Procédé selon la revendication 3, **caractérisé en ce que** sur la base de l'ADNc obtenue par transcriptase inverse (RT), on amplifie dans une première étape PCR la totalité de l'ADNc de β hCG au moyen d'au moins une première paire d'amorces, la première paire d'amorces s'hybridant aussi bien avec l'ADNc de β5 - hCG, de β8 - hCG, de β3 - hCG que de β7 - hCG et de β6 - hCG et d'un ARNm dont l'ADNc présente une séquence selon SEQ ID N° 7, et dans une seconde étape PCR successive on amplifie spécifiquement au moyen d'au moins une troisième amorce l'ADNc de β7 - hCG et/ou de β6 - hCG et/ou d'un ARNm dont l'ADNc présente une séquence selon SEQ ID N° 7, la troisième amorce s'hybridant spécifiquement avec l'ADNc de β7 - hCG et de β6 - hCG et d'un ARNm dont l'ADNc présente une séquence selon SEQ ID N° 7, mais non pas avec l'ADNc de β5 - hCG, de β8 - hCG et de β3 - hCG.

5. Procédé selon la revendication 4, **caractérisé en ce que** dans la seconde étape PCR on amplifie spécifiquement l'ADNc de β5 - hCG et/ou de β8 - hCG et/ou de β3 - hCG en supplément au moyen d'au moins une quatrième amorce, la quatrième amorce s'hybridant spécifiquement avec l'ADNc de β5 - hCG, de β8 - hCG et de β3 - hCG, mais non pas avec l'ADNc de β7 - hCG et de β6 - hCG et d'un ARNm dont l'ADNc présente une séquence selon SEQ ID N° 7.

6. Procédé selon la revendication 4 ou 5, **caractérisé en ce que** utilise en tant que première paire d'amorces les paires d'oligonucléotides parmi le groupe comprenant les séquences selon SEQ ID N° 1 et SEQ ID N° 2, selon SEQ ID N° 1 et SEQ ID N° 11 ou SEQ ID N° 14 et SEQ ID N° 2 dans la première étape PCR, et en tant que troisième amorce un oligonucléotide parmi le groupe comprenant les séquences selon SEQ ID N° 3, SEQ ID N° 9, SEQ ID N° 10, SEQ ID N° 13 et SEQ ID N° 16 dans la seconde étape PCR.

7. Procédé selon la revendication 5 ou 6, **caractérisé en ce que** dans la seconde étape PCR on utilise en tant que quatrième amorce un oligonucléotide parmi le groupe comprenant les séquences selon SEQ ID N° 4, SEQ ID N° 8, SEQ ID N° 12 et SEQ ID N° 15.

8. Procédé selon l'une des revendications 4 à 7, **caractérisé en ce qu'**au moins une amorce est marquée par fluorescence.

9. Procédé selon la revendication 8, **caractérisé en ce qu'**une amorce de la première paire d'amorces, la troisième amorce et le cas échéant la quatrième amorce sont pourvues de marqueurs fluorescents qui se distinguent entre eux de par leurs spectres d'absorption et/ou d'émission.

10. Utilisation du procédé selon l'une des revendications 1 à 9 pour le diagnostic prospectif ou rétrospectif d'une réceptivité endométriale pour une implantation d'embryon.

11. Utilisation selon la revendication 10, **caractérisée en ce que** l'on prélève du sang périphérique ou du tissu de l'endomètre ou du cervix chez la patiente et **en ce que** l'analyse de l'expression de l'ARNm s'effectue dans cet échantillon de sang ou de tissu, et à partir du niveau de l'expression déterminée de l'ARNm de β7 - hCG et/ou de β6 - hCG et/ou d'un ARNm dont l'ADNc présente une séquence selon SEQ ID N° 7, on peut tirer des conclusions sur la réceptivité de l'utérus vis-à-vis d'un embryon dans le cycle actuel.

12. Utilisation selon la revendication 10, **caractérisée en ce que** l'analyse de l'expression de l'ARNm s'effectue dans un échantillon de sang menstruel d'une patiente, et à partir du niveau de l'expression déterminée de l'ARNm de β7 - hCG et/ou de β6 - hCG et/ou d'un ARNm dont l'ADNc présente une séquence selon SEQ ID N° 7, dans le cycle précédent, on effectue des prévisions sur la réceptivité potentielle de l'utérus vis-à-vis d'un embryon dans le cycle suivant.

13. Utilisation d'une amorce pour la détermination de la réceptivité de la muqueuse utérine pour l'implantation d'un embryon, l'amorce présentant une séquence d'amorce selon SEQ ID N° 3 ou SEQ ID N° 4 ou l'une parmi les SEQ ID N° 8 à SEQ ID N° 16.

14. Utilisation d'un kit de diagnostic pour la détermination de la réceptivité de la muqueuse utérine pour l'implantation d'un embryon au moyen d'une RT - PCR quantitative, le kit de diagnostic comprenant une quantité respective de
a.) Oligo-dT,
b.) l'enzyme de la transcriptase inverse,
c.) une paire d'amorces choisie parmi le groupe comprenant des séquences selon SEQ ID N° 1 et SEQ ID N° 2, selon SEQ ID N° 1 et SEQ ID N° 11 ou SEQ ID N° 14 et SEQ ID N° 2,
d.) une ADN polymérase résistante à plus de 80 °C,
e.) un tampon de réaction.

15. Utilisation d'un kit de diagnostic selon la revendication 14, **caractérisée en ce qu'**il comprend une quantité d'une quatrième amorce choisie parmi le groupe comprenant des séquences selon SEQ ID N° 4, SEQ ID N° 8, SEQ ID N° 12 et SEQ ID N° 15.

16. Utilisation d'un kit de diagnostic selon l'une des revendications 14 ou 15, **caractérisée en ce qu'**au moins une amorce est marquée de fluorescence.

17. Utilisation d'un kit de diagnostic selon la revendication 16, **caractérisée en ce qu'**une amorce de la première paire d'amorces, la troisième amorce et le cas échant la quatrième amorce sont pourvues de marqueurs fluorescents qui se distinguent entre eux de par leurs spectres d'absorption et/ou d'émission.

18. Utilisation d'un kit de diagnostic selon l'une des revendications 14 à 17, **caractérisée en ce qu'**il comprend en tant que standard une quantité définie d'ARNm ou d'ADNc de β5 - hCG et/ou de β7 - hCG.

19. Utilisation d'un kit de diagnostic selon l'une des revendications 14 à 18 pour le diagnostic prospectif ou rétrospectif de la réceptivité de la muqueuse utérine pour l'implantation de l'embryon.

20. Utilisation d'une ADNc pour la détermination de la réceptivité de la muqueuse utérine pour l'implantation d'un embryon, l'ADNc présentant une séquence génétique qui relève de SEQ ID N° 7 et/ou une séquence génétique selon SEQ ID N° 5 et/ou SEQ ID N° 6 et/ou une séquence génétique codant pour une protéine de la séquence d'acides aminés selon SEQ ID N° 17 ou 18 en tant que marqueur pour le diagnostic prospectif ou rétrospectif de la réceptivité de la muqueuse utérine pour l'implantation de l'embryon.


### Sequenzprotokoll - Sequence Listing

<110> Universität Leipzig
   <120> Verfahren und Mittel zur Bestimmung von bestimmten Zuständen bzw. Veränderungen im Uterusepithel und in Epithelien anderer Organe
   <130> 401P04PCT
   <150> DE10260556.4
   <151> 2002-12-21
   <150> DE10325637.7
   <151> 2003-06-06
   <150> DE10325636.9
   <151> 2003-06-06
<160> 18
<210> 1
   <211> 20
   <212> DNA
   <213> artificial
   <220>
   <223> Primer 1 (ßhCG gesamt)
<301> Lindholm-Miller A.K. Labenz C.J., Ramey J., Bedows E., Ruddon R.W.
   <302> Human Chorionic Gonadotropin-ß-Gene Expression in First Trimester Placenta
   <303> Endocrinology
   <304> 138
   <305> 12
   <306> 5459-5465
   <307> 1997
<400>
   tcacttcacc gtggtctccg 20
<210> 2
   <211> 20
   <212> DNA
   <213> artificial
<220>
   <223> Primer 2 (ßhCG gesamt)
<301> Lindholm-Miller A.K. Labenz C.J., Ramey J., Bedows E., Ruddon R.W.
   <302> Human Chorionic Gonadotropin-ß-Gene Expression in First Trimester Placenta
   <303> Endocrinology
   <304> 138
   <305> 12
   <306> 5459-5465
   <307> 1997
<400> 2
   tgcagcacgc gggtcatggt 20
<210>
   <211> 23
   <212> DNA
   <213> artificial
<220>
   <223> Primer 3 (ßhCG ß7, ß6, ß6e)
<400> 3
   cactgagggg agaggactgg ggt 23
<210> 4
   <211> 23
   <212> DNA
   <213> artificial
<220>
   <223> Primer 4 (ßhCG ß5, ß8, ß3)
<400> 4
   cagtgagagg agagggctgg ggc 23
<210> 5
   <211> 861
   <212> DNA
   <213> human
<220>
   <223> ßhCG ß7 cDNA-Sequenz
<400>5
   agcactttcc tcgggtcacg gcctcctcct ggttcccaag accccaccat aggcagaggc 60
   aggccttcct acaccctact ctctgtgcct ccagcctcga ctagtcccta gcactcgacg 120
   actgagtctc agaggtcact tcaccgtggt ctccgcctca tccttggcgc tagaccactg 180
   aggggagagg actggggtgc tccgctgagc cactcctgtg cctccctggc cttgtctact 240
   tctcgccccc cgaagggtta gtgtccagct cactccagca tcctacaacc tcctggtggc 300
   cttgacgccc ccacaaaccc gaggtataaa gccaggtaca ccaggcaggg gacgcaccaa 360
   ggatggagat gttccagggg ctgctgctgt tgctgctgct gagcatgggc gggacatggg 420
   catccaagga gatgcttcgg ccacggtgcc gccccatcaa tgccaccctg gctgtggaga 480
   aggagggctg ccccgtgtgc atcaccgtca acaccaccat ctgtgccggc tactgcccca 540
   ccatgacccg cgtgctgcag gggtcctgcc cggccctgcc tcaggtggtg tgcaactacc 600
   gcgatgtgcg cttcgagtcc atccggctcc ctggctgccc gcgcggcgtg aaccccgtgg 660
   tctcctacgc cgtggctctc agctgtcaat gtgcactctg ccgccgcagc accactgact 720
   gcgggggtcc caaggaccac cccttgacct gtgatgaccc ccgcttccag gcctcctctt 780
   cctcaaaggc ccctcccccc agccttccaa gtccatcccg actcccgggg ccctcggaca 840
   ccccgatcct cccacaataa a 861
<210> 6
   <211> 861
   <212> DNA
   <213> human
<220>
   <223> ßhCG ß6 cDNA-Sequenz
<400>6
   agcactttcc tcgggtcacg gcctcctcct ggttcccaag accccaccat aggcagaggc 60
   aggccttcct acaccctact ctctgtgcct ccagcctcga ctagtcccta acactcgacg 120
   actgagtctc agaggtcact tcaccgtggt ctccgcctca tccttggcgc tagaccactg 180
   aggggagagg actggggtgc tccgctgagc cactcctgtg cctccctggc cttgtctact 240
   tctcgccccc cgaagggtta gtgtcgagct cactccagca tcctacaacc tcctggtggc 300
   cttgccgccc ccacaacccc gaggtatgaa gccaggtaca ccaggcaggg gacgcaccaa 360
   ggatggagat gttccagggg ctgctgctgt tgctgctgct gagcatgggc gggacatggg 420
   catccaagga gccacttcgg ccacggtgcc gccccatcaa tgccaccctg gctgtggaga 480
   aggagggctg ccccgtgtgc atcaccgtca acaccaccat ctgtgccggc tactgcccca 540
   ccatgacccg cgtgctgcag gggtcctgcc cggccctgcc tcaggtggtg tgcaactacc 600
   gcgatgtgcg cttcgagtcc atccggctcc ctggctgccc gcgcggcgtg aaccccgtgg 660
   tctcctacgc cgtggctctc agctgtcaat gtgcactctg ccgccgcagc accactgact 720
   gcgggggtcc caaggaccac cccttgacct gtgatgaccc ccgcttccag gcctcctctt 780
   cctcaaaggc ccctcccccc agccttccaa gtccatcccg actcccgggg ccctcggaca 840
   ccccgatcct cccacaataa a 861
<210> 7
   <211> 861
   <212> DNA
   <213> human
<220>
   <223> ßhCG ß6e cDNA-Sequenz
<400>7
   agcactttyc tcgggtcacg gcctcctcct ggttcccaag accccaccat aggcagaggc   60
   aggccttcct acaccctact ctctgtgcct ccagcctcga ctagtcccta rcactcgacg  120
   actgagtctc agaggtcact tcaccgtggt ctccgcctca tccttggygc tagaccactg  180
   aggggagagg actggggtgc tccgctgagc cactcctgtg cctccctggc cttgtctact  240
   tctcgccccc cgaagggtta gtgtcsagct cactccagca tcctacaacc tcctggtggc  300
   cttgmcgccc ccacaamccc gaggtatraa gccaggtaca ccaggcaggg gacgcaccaa  360
   ggatggagat gttccagggg ctgctgctgt tgctgctgct gagcatgggc gggacatggg  420
   catccargga gmyrcttcgg ccacggtgcc gccccatcaa tgccaccctg gctgtggaga  480
   aggagggctg ccccgtgtgc atcaccgtca acaccaccat ctgtgccggc tactgcccca  540
   ccatgacccg cgtgctgcag ggggtcctgc cggccctgcc tcaggtggtg tgcaactacc  600
   gcgatgtgcg cttcgagtcc atccggctcc ctggctgccc gcgcggcgtg aaccccgtgg  660
   tctcctacgc cgtggctctc agctgtcaat gtgcactctg ccgccgcagc accactgact  720
   gcgggggtcc caaggaccac cccttgacct gtgatgaccc ccgcttccag gcctcctctt  780
   cctcaaaggc cctccccccc agccttccaa gtccatcccg actcccgggg ccctcggaca  840
   ccccgatcct cccacaataa a                                            861
<210> 8
   <211> 20
   <212> DNA
   <213> artificial
<220>
   <223> Primer 8 (ßhCG ß5, ß8, ß3)
<400> 8
   catgggcatc caaggagccg 20
<210> 9
   <211> 20
   <212> DNA
   <213> artificial
<220>
   <223> Primer 9 (ßhCG ß6)
<400> 9
   catgggcatc caaggagcca 20
<210> 10
   <211> 20
   <212> DNA
   <213> artificial
<220>
   <223> Primer 10 (ßhCG ß7, ß6e)
<400> 10
   catgggcatc cagggagatg 20
<210> 11
   <211> 17
   <212> DNA
   <213> artificial
<220>
   <223> Primer 11 (Gesamt- ßhCG)
<400> 11
   tcggggtgtc cgagggc 17
<210> 12
   <211> 20
   <212> DNA
   <213> artificial
<220>
   <223> Primer 12 (ßhCG ß5, ß8, ß3)
<400> 12
   gatgaccccc gcttccagga 20
<210> 13
   <211> 20
   <212> DNA
   <213> artificial
<220>
   <223> Primer 13 (ßhCG ß7, ß6)
<400> 13
   gatgaccccc cgttccaggc 20
<210> 14
   <211> 17
   <212> DNA
   <213> artificial
<220>
   <223> Primer 14 (Gesamt-ßhCG)
<400> 14
   tcgggtcacg gcctcct 17
<210> 15
   <211> 22
   <212> DNA
   <213> artificial
<220>
   <223> Primer 15 (ßhCG ß5, ß8, ß3)
<400> 15
   acggcctcct cctggctccc ag 22
<210> 16
   <211> 22
   <212> DNA
   <213> artificial
<220>
   <223> Primer 16 (ßhCG ß7, ß6, ß6e)
<400> 16
   acggcctcct cctggttccc aa 22
<210> 17
   <211>..165
   <212> PRT
   <213> human
<220>
   <223> ßhCG ß6eI (with Lys in Pos 2)
<400> 17
<210> 18
   <211>..165
   <212> PRT
   <213> human
<220>
   <223> ßhCG ß6eII (with Arg in Pos 2)
<400> 18